# EUROPEAN PATENT SPECIFICATION

(11) **EP 3 212 662 B1**
(45) Date of publication and mention of the grant of the patent: **08.04.2020**
(21) Application number: 15791844.2
(22) Date of filing: 27.10.2015
(51) Int. Cl.: C12N 9/54

(54) **SERINE PROTEASES**
SERINPROTEASEN
SÉRINES PROTÉASES

(30) Priority: 27.10.2014 US 201462069192 P
(43) Date of publication of application: 06.09.2017
(73) Proprietor: Danisco US Inc., Palo Alto, California 94304 (US)
(72) Inventor: KOLKMAN, Marc, NL-2341 PA Oegstgeest (NL); KELLETT-SMITH, Anja Hemmingsen, DK-8000 Århus C (DK); MEJLDAL, Rie, DK-8752 Østbirk (DK); BABE, Lilia Maria, Palo Alto, CA 94304 (US)
(74) Representative: Mewburn Ellis LLP
(86) International application number: PCT/US2015/057502
(87) International publication number: WO 2016/069552

(56) References cited:
- EP-A1- 0 405 901
- US-A- 5 677 163
- DATABASE NCBI [Online] 11 June 2014 (2014-06-11), "Peptidase S8 (Bacillus aurantiacus)", XP002754206, Database accession no. WP026691049 cited in the application
- PHILIP N. BRYAN: "Protein engineering of subtilisin", BIOCHIMICA ET BIOPHYSICA ACTA. PROTEIN STRUCTURE AND MOLECULAR ENZYMOLOGY, vol. 1543, no. 2, 1 December 2000 (2000-12-01), pages 203-222, XP55251064, AMSTERDAM; NL ISSN: 0167-4838, DOI: 10.1016/S0167-4838(00)00235-1

## Description

The present disclosure relates to serine proteases cloned from *Bacillus spp.,* and variants thereof. Compositions containing the serine proteases are suitable for use in cleaning fabrics and hard surfaces, as well as in a variety of industrial applications.

Serine proteases are enzymes (EC No. 3.4.21) possessing an active site serine that initiates hydrolysis of peptide bonds of proteins. There are two broad categories of serine proteases, based on their structure: chymotrypsin-like (trypsin-like) and subtilisin-like. The prototypical subtilisin (EC No. 3.4.21.62) was initially obtained from *Bacillus subtilis.* Subtilisins and their homologues are members of the S8 peptidase family of the MEROPS classification scheme. Members of family S8 have a catalytic triad in the order Asp, His and Ser in their amino acid sequence.

Although serine proteases have long been known in the art of industrial enzymes, there remains a need for further serine proteases that are suitable for particular conditions and uses.

WO 2016/145428 discloses compositions comprising LG12-clade protease variants and methods of use.

EP 0405901 A1 discloses enzymatic detergent compositions comprising subtilisin variants for use in cleaning products.

US 5 677 163 A discloses Bacillus sp., derived subtilisin enzymes for use in cleaning compositions.

The present compositions and methods relate to recombinant serine proteases cloned from *Bacillus spp.,* and variants thereof as defined in the claims. Compositions containing the serine proteases are suitable for use in cleaning fabrics and hard surfaces, as well as in a variety of industrial applications.

In a first aspect the invention provides a subtilisin or recombinant polypeptide, or active fragment thereof, wherein said subtilisin, polypeptide or fragment comprises a XQXVPYGXEXIGAXDVQQND (SEQ ID NO:32) motif, wherein X is any amino acid, and wherein said subtilisin, polypeptide or fragment comprises an amino acid sequence having at least 85% amino acid sequence identity to the amino acid sequence SEQ ID NO: 3 or 6, and wherein said subtilisin, polypeptide or fragment has protease activity.

In some embodiments the subtilisin, polypeptide or fragment has protease activity on a dimethylcasein substrate.

In a second aspect the invention provides a composition comprising: i) a surfactant; and ii) the subtilisin, polypeptide or fragment of the first aspect of the invention.

In some embodiments the surfactant is selected from the group consisting of an anionic surfactant, a cationic surfactant, a zwitterionic surfactant, an ampholytic surfactant, a semi-polar non-ionic surfactant, and a combination thereof.

In some embodiments:
a) said composition further comprises at least one calcium ion and/or zinc ion; at least one stabilizer; from about 0.001 % to about 1.0 weight % of said subtilisin, polypeptide or fragment of any one of Claims 1-2; at least one bleaching agent; at least one adjunct ingredient; and/or one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, additional serine proteases, and combinations thereof;
b) said composition contains phosphate or is phosphate-free and/or contains borate or is borate-free; and/or
c) said composition is formulated at a pH of from 8 to 12.

In a third aspect the invention provides a method of cleaning, comprising: contacting a surface or an item in need of cleaning with the subtilisin, polypeptide or fragment of the first aspect of the invention or the composition of the second aspect of the invention; and optionally further comprising the step of rinsing said surface or item after contacting said surface or item with said subtilisin, recombinant polypeptide, or fragment thereof, or after contacting said surface or item with said composition.

In a fourth aspect the invention provides a polynucleotide comprising a nucleic acid sequence encoding the subtilisin, polypeptide or fragment of the first aspect of the invention.

In some embodiments the polynucleotide comprises a nucleic acid sequence having at least 85% identity to the nucleic acid sequence of SEQ ID NO:1 or 4.

In a fifth aspect the invention provides an expression vector comprising the polynucleotide of the fourth aspect of the invention.

In a sixth aspect the invention provides a host cell comprising the vector of the fifth aspect of the invention.

In some embodiments the host cell is of a species selected from *Bacillus spp., Streptomyces spp., Escherichia spp., Aspergillus spp., Trichoderma spp., Pseudomonas spp., Corynebacterium spp., Saccharomyces spp.,* and *Pichia spp.*

In a seventh aspect the invention provides a method for producing the subtilisin, polypeptide or fragment of the first aspect of the invention, comprising:
(a) stably transforming the host cell of the sixth aspect of the invention with the expression vector of the fifth aspect of the invention;
(b) cultivating said transformed host cell under conditions suitable for said host cell to produce said subtilisin or polypeptide; and
(c) recovering said subtilisin or polypeptide.

In some embodiments said expression vector comprises a heterologous polynucleotide sequence encoding a heterologous pro-peptide, or one or both of a heterologous promoter and a polynucleotide sequence encoding a heterologous signal peptide.

### BRIEF DESCRIPTION OF THE DRAWINGS

Figure 1 provides a plasmid map for expression of BspM00695 protease.
Figure 2 provides a plasmid map for expression of BspM04284 protease.
Figure 3A-C provides a MUSCLE multiple sequence alignment of subtilisins including BspM04284, BspM00695, and BspZ00258.
Figure 4 provides a phylogenetic tree of subtilisins including BspM04284, BspM00695, and BspZ00258.

Described are compositions and methods relating to recombinant serine proteases from *Bacillus* species. The compositions and methods are based, in part, on the observation that recombinant BspM04284 and BspM00695have protease activity in the presence of a surfactant, in basic reaction conditions, and at elevated temperatures. These features of BspM04284 and BspM00695 make these proteases well suited for use in cleansing fabrics and hard surfaces, as well as in textile, leather and feather processing. The new proteases are also well suited to inclusion in compositions for protein degradation, including but not limited to laundry and dish washing detergents.

Prior to describing the present compositions and methods in detail, the following terms are defined for clarity. Terms and abbreviations not defined should be accorded their ordinary meaning as used in the art. Unless defined otherwise herein, all technical and scientific terms used herein have the same meaning as commonly understood by one of ordinary skill in the art. Unless otherwise indicated, the practice of the present disclosure involves conventional techniques commonly used in molecular biology, protein engineering, and microbiology. Although any methods and materials similar or equivalent to those described herein find use in the practice of the present disclosure, some suitable methods and materials are described herein. The terms defined immediately below are more fully described by reference to the Specification as a whole.

As used herein, the singular "a," "an" and "the" includes the plural unless the context clearly indicates otherwise. Unless otherwise indicated, nucleic acid sequences are written left to right in 5' to 3' orientation; and amino acid sequences are written left to right in amino to carboxy orientation. It is to be understood that this disclosure is not limited to the particular methodology, protocols, and reagents described herein, absent an indication to the contrary.

It is intended that every maximum numerical limitation given throughout this Specification includes every lower numerical limitation, as if such lower numerical limitations were expressly written herein. Every minimum numerical limitation given throughout this Specification will include every higher numerical limitation, as if such higher numerical limitations were expressly written herein. Every numerical range given throughout this Specification will include every narrower numerical range that falls within such broader numerical range, as if such narrower numerical ranges were all expressly written herein.

As used herein in connection with a numerical value, the term "about" refers to a range of +/- 0.5 of the numerical value, unless the term is otherwise specifically defined in context. For instance, the phrase a "pH value of about 6" refers to pH values of from 5.5 to 6.5, unless the pH value is specifically defined otherwise.

As used herein, the terms "protease" and "proteinase" refer to an enzyme that has the ability to break down proteins and peptides. A protease has the ability to conduct "proteolysis," by hydrolysis of peptide bonds that link amino acids together in a peptide or polypeptide chain forming the protein. This activity of a protease as a protein-digesting enzyme is referred to as "proteolytic activity." Many well-known procedures exist for measuring proteolytic activity. For example, proteolytic activity may be ascertained by comparative assays that analyze the respective protease's ability to hydro lyze a suitable substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (See e.g., WO99/34011 and US 6,376,450). The pNA peptidyl assay (See e.g., Del Mar et al., Anal Biochem, 99:316-320, 1979) also finds use in determining the active enzyme concentration. This assay measures the rate at which p-nitroaniline is released as the enzyme hydrolyzes a soluble synthetic substrate, such as succinyl-alanine-alanine-proline-phenylalanine-p-nitroanilide (suc-AAPF-pNA). The rate of production of yellow color from the hydrolysis reaction is measured at 410 nanometers (nm) on a spectrophotometer and is proportional to the active enzyme concentration. In addition, absorbance measurements at 280 nm can be used to determine the total protein concentration in a sample of purified protein. The activity on substrate/protein concentration gives the enzyme specific activity.

The term "variant," with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

As used herein, "the genus Bacillus" includes all species within the genus "Bacillus," as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. gibsonii,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus", or B. polymyxa,* which is now *"Paenibacillus polymyxa".* The production of resistant endospores under stressful environmental conditions is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

As used herein, the term "mutation" refers to changes made to a reference amino acid or nucleic acid sequence. It is intended that the term encompass substitutions, insertions and deletions.

As used herein, the term "vector" refers to a nucleic acid construct used to introduce or transfer nucleic acid(s) into a target cell or tissue. A vector is typically used to introduce foreign DNA into a cell or tissue. Vectors include plasmids, cloning vectors, bacteriophages, viruses (e.g., viral vector), cosmids, expression vectors, shuttle vectors, and the like. A vector typically includes an origin of replication, a multicloning site, and a selectable marker. The process of inserting a vector into a target cell is typically referred to as transformation. The present invention includes, in some embodiments, a vector that comprises a DNA sequence encoding a serine protease polypeptide (e.g., precursor or mature serine protease polypeptide) that is operably linked to a suitable prosequence (e.g., secretory, signal peptide sequence, etc.) capable of effecting the expression of the DNA sequence in a suitable host, and the folding and translocation of the recombinant polypeptide chain.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction. Transformation refers to the genetic alteration of a cell which results from the uptake, optional genomic incorporation, and expression of genetic material (e.g., DNA).

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

As used herein the term "gene" refers to a polynucleotide (e.g., a DNA segment), that encodes a polypeptide and includes regions preceding and following the coding regions. In some instances a gene includes intervening sequences (introns) between individual coding segments (exons).

As used herein, "recombinant" when used with reference to a cell typically indicates that the cell has been modified by the introduction of a foreign nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) that has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA in vitro and transfer of the recombinant DNA into cells where it maybe expressed or propagated, thereby producing a recombinant polypeptide. "Recombination" and "recombining" ofpolynucleotides or nucleic acids refer generally to the assembly or combining of two or more nucleic acid or polynucleotide strands or fragments to generate a new polynucleotide or nucleic acid.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

The terms "host strain" and "host cell" refer to a suitable host for an expression vector comprising a DNA sequence of interest.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code. Mutations can be named by the one letter code for the parent amino acid, followed by a position number and then the one letter code for the variant amino acid. For example, mutating glycine (G) at position 87 to serine (S) is represented as "G087S" or "G87S". When describing modifications, a position followed by amino acids listed in parentheses indicates a list of substitutions at that position by any of the listed amino acids. For example, 6(L,I) means position 6 can be substituted with a leucine or isoleucine. At times, in a sequence, a slash (/) is used to define substitutions, e.g. F/V, indicates that the particular position may have a phenylalanine or valine at that position.

A "prosequence" or "propeptide sequence" refers to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Bacterial serine proteases are often expressed as pro-enzymes.

The terms "signal sequence" and "signal peptide" refer to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (e.g., polypeptides cleaved therefrom to leave the mature form of a protein or peptide).

The term "wild-type" in reference to an amino acid sequence or nucleic acid sequence indicates that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild-type sequence).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question. To exemplify, "proteases derived from Bacillus" refers to those enzymes having proteolytic activity that are naturally produced by Bacillus, as well as to serine proteases like those produced by Bacillus sources but which through the use of genetic engineering techniques are produced by other host cells transformed with a nucleic acid encoding the serine proteases.

The term "identical" in the context of two polynucleotide or polypeptide sequences refers to the nucleic acids or amino acids in the two sequences that are the same when aligned for maximum correspondence, as measured using sequence comparison or analysis algorithms described below and known in the art.

As used herein, "% identity" or percent identity" or "PID" refers to protein sequence identity. Percent identity may be determined using standard techniques known in the art. Useful algorithms include the BLAST algorithms (See, Altschul et al., J Mol Biol, 215:403-410, 1990; and Karlin and Altschul, Proc Natl Acad Sci USA, 90:5873-5787, 1993). The BLAST program uses several search parameters, most of which are set to the default values. The NCBI BLAST algorithm finds the most relevant sequences in terms of biological similarity but is not recommended for query sequences of less than 20 residues (Altschul et al., Nucleic Acids Res, 25:3389-3402, 1997; and Schaffer et al., Nucleic Acids Res, 29:2994-3005, 2001). Exemplary default BLAST parameters for a nucleic acid sequence searches include: Neighboring words threshold = 11; E-value cutoff = 10; Scoring Matrix = NUC.3.1 (match = 1, mismatch = -3);Gap Opening = 5; and Gap Extension = 2. Exemplary default BLAST parameters for amino acid sequence searches include: Word size = 3; E-value cutoff = 10; Scoring Matrix = BLOSUM62; Gap Opening = 11; and Gap extension = 1. A percent (%) amino acid sequence identity value is determined by the number of matching identical residues divided by the total number of residues of the "reference" sequence including any gaps created by the program for optimal/maximum alignment. BLAST algorithms refer to the "reference" sequence as the "query" sequence.

As used herein, "homologous proteins" or "homologous proteases" refers to proteins that have distinct similarity in primary, secondary, and/or tertiary structure. Protein homology can refer to the similarity in linear amino acid sequence when proteins are aligned. Homologous search of protein sequences can be done using BLASTP and PSI-BLAST from NCBI BLAST with threshold (E-value cut-off) at 0.001 (Altschul SF, Madde TL, Shaffer AA, Zhang J, Zhang Z, Miller W, Lipman DJ. Gapped BLAST and PSI BLAST a new generation of protein database search programs. Nucleic Acids Res 1997 Set 1; 25(17):3389-402). Using this information, proteins sequences can be grouped. A phylogenetic tree can be built using the amino acid sequences. Amino acid sequences can be entered in a program such as the Vector NTI Advance suite and a Guide Tree can be created using the Neighbor Joining (NJ) method (Saitou and Nei, Mol Biol Evol, 4:406-425, 1987). The tree construction can be calculated using Kimura's correction for sequence distance and ignoring positions with gaps. A program such as AlignX can display the calculated distance values in parenthesis following the molecule name displayed on the phylogenetic tree.

Another useful algorithm for comparison of multiple protein sequences is the MUSCLE program from Geneious software (Biomatters Ltd.) (Robert C. Edgar. MUSCLE: multiple sequence alignment with high accuracy and high throughput Nucl. Acids Res. (2004) 32 (5): 1792-1797).

Understanding the homology between molecules can reveal the evolutionary history of the molecules as well as information about their function; if a newly sequenced protein is homologous to an already characterized protein, there is a strong indication of the new protein's biochemical function. The most fundamental relationship between two entities is homology; two molecules are said to be homologous if they have been derived from a common ancestor. Homologous molecules, or homologs, can be divided into two classes, paralogs and orthologs. Paralogs are homologs that are present within one species. Paralogs often differ in their detailed biochemical functions. Orthologs are homologs that are present within different species and have very similar or identical functions. A protein superfamily is the largest grouping (clade) of proteins for which common ancestry can be inferred. Usually this common ancestry is based on sequence alignment and mechanistic similarity. Superfamilies typically contain several protein families which show sequence similarity within the family. The term "protein clan" is commonly used for protease superfamilies based on the MEROPS protease classification system.

The CLUSTAL W algorithm is another example of a sequence alignment algorithm (See, Thompson et al., Nucleic Acids Res, 22:4673-4680, 1994). Default parameters for the CLUSTAL W algorithm include: Gap opening penalty = 10.0; Gap extension penalty = 0.05; Protein weight matrix = BLOSUM series; DNA weight matrix = IUB; Delay divergent sequences % = 40; Gap separation distance = 8; DNA transitions weight = 0.50; List hydrophilic residues = GPSNDQEKR; Use negative matrix = OFF; Toggle Residue specific penalties = ON; Toggle hydrophilic penalties = ON; and Toggle end gap separation penalty = OFF. In CLUSTAL algorithms, deletions occurring at either terminus are included. For example, a variant with a five amino acid deletion at either terminus (or within the polypeptide) of a polypeptide of 500 amino acids would have a percent sequence identity of 99% (495/500 identical residues × 100) relative to the "reference" polypeptide. Such a variant would be encompassed by a variant having "at least 99% sequence identity" to the polypeptide.

A nucleic acid or polynucleotide is "isolated" when it is at least partially or completely separated from other components, including but not limited to for example, other proteins, nucleic acids, cells, etc. Similarly, a polypeptide, protein or peptide is "isolated" when it is at least partially or completely separated from other components, including but not limited to for example, other proteins, nucleic acids, cells, etc. On a molar basis, an isolated species is more abundant than are other species in a composition. For example, an isolated species may comprise at least about 60%, about 65%, about 70%, about 75%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (i.e., contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

As used herein, the term "functional assay" refers to an assay that provides an indication of a protein's activity. In some embodiments, the term refers to assay systems in which a protein is analyzed for its ability to function in its usual capacity. For example, in the case of a protease, a functional assay involves determining the effectiveness of the protease to hydrolyze a proteinaceous substrate.

The term "cleaning activity" refers to a cleaning performance achieved by a serine protease polypeptide or reference protease under conditions prevailing during the proteolytic, hydrolyzing, cleaning, or other process of the disclosure. In some embodiments, cleaning performance of a serine protease polypeptide or reference protease may be determined by using various assays for cleaning one or more various enzyme sensitive stains on an item or surface (e.g., a stain resulting from food, grass, blood, ink, milk, oil, and/or egg protein). Cleaning performance of a variant or reference protease can be determined by subjecting the stain on the item or surface to standard wash condition(s) and assessing the degree to which the stain is removed by using various chromatographic, spectrophotometric, or other quantitative methodologies. Exemplary cleaning assays and methods are known in the art and include, but are not limited to those described in WO99/34011 and US 6,605,458, as well as those cleaning assays and methods included in the Examples provided below.

The term "cleaning effective amount" of a serine protease polypeptide or reference protease refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (e.g., granular, tablet, bar) composition is required, etc.

The term "cleaning adjunct material" refers to any liquid, solid, or gaseous material included in cleaning composition other than a serine protease polypeptide of the disclosure. In some embodiments, the cleaning compositions of the present disclosure include one or more cleaning adjunct materials. Each cleaning adjunct material is typically selected depending on the particular type and form of cleaning composition (e.g., liquid, granule, powder, bar, paste, spray, tablet, gel, foam, or other composition). Preferably, each cleaning adjunct material is compatible with the protease enzyme used in the composition.

Cleaning compositions and cleaning formulations include any composition that is suited for cleaning, bleaching, disinfecting, and/or sterilizing any object, item, and/or surface. Such compositions and formulations include, but are not limited to for example, liquid and/or solid compositions, including cleaning or detergent compositions (e.g., liquid, tablet, gel, bar, granule, and/or solid laundry cleaning or detergent compositions and fine fabric detergent compositions; hard surface cleaning compositions and formulations, such as for glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including hand or manual dishwashing compositions (e.g., "hand" or "manual" dishwashing detergents) and automatic dishwashing compositions (e.g., "automatic dishwashing detergents"). Single dosage unit forms also find use with the present invention, including but not limited to pills, tablets, gelcaps, or other single dosage units such as pre-measured powders or liquids.

Cleaning composition or cleaning formulations, as used herein, include, unless otherwise indicated, granular or powder-form all-purpose or heavy-duty washing agents, especially cleaning detergents; liquid, granular, gel, solid, tablet, paste, or unit dosage form all-purpose washing agents, especially the so-called heavy-duty liquid (HDL) detergent or heavy-duty dry (HDD) detergent types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types. In some embodiments, granular compositions are in "compact" form; in some embodiments, liquid compositions are in a "concentrated" form.

As used herein, "fabric cleaning compositions" include hand and machine laundry detergent compositions including laundry additive compositions and compositions suitable for use in the soaking and/or pretreatment of stained fabrics (e.g., clothes, linens, and other textile materials).

As used herein, "non-fabric cleaning compositions" include non-textile (i.e., non-fabric) surface cleaning compositions, including, but not limited to for example, hand or manual or automatic dishwashing detergent compositions, oral cleaning compositions, denture cleaning compositions, contact lens cleaning compositions, wound debridement compositions, and personal cleansing compositions.

As used herein, the term "detergent composition" or "detergent formulation" is used in reference to a composition intended for use in a wash medium for the cleaning of soiled or dirty objects, including particular fabric and/or non-fabric objects or items. Such compositions of the present disclosure are not limited to any particular detergent composition or formulation. Indeed, in some embodiments, the detergents of the disclosure comprise at least one serine protease polypeptide of the disclosure and, in addition, one or more surfactants, transferase(s), hydrolytic enzymes, oxido reductases, builders (e.g., a builder salt), bleaching agents, bleach activators, bluing agents, fluorescent dyes, caking inhibitors, masking agents, enzyme activators, antioxidants, and/or solubilizers. In some instances, a builder salt is a mixture of a silicate salt and a phosphate salt, preferably with more silicate (e.g., sodium metasilicate) than phosphate (e.g., sodium tripolyphosphate). Some compositions of the disclosure, such as, but not limited to, cleaning compositions or detergent compositions, do not contain any phosphate (e.g., phosphate salt or phosphate builder).

As used herein, the term "bleaching" refers to the treatment of a material (e.g., fabric, laundry, pulp, etc.) or surface for a sufficient length of time and/or under appropriate pH and/or temperature conditions to effect a brightening (i.e., whitening) and/or cleaning of the material. Examples of chemicals suitable for bleaching include, but are not limited to, for example, ClO₂, H₂O₂, peracids, NO₂, etc.

As used herein, "wash performance" of a protease (e.g., a serine protease polypeptide of the disclosure) refers to the contribution of a serine protease polypeptide to washing that provides additional cleaning performance to the detergent as compared to the detergent without the addition of the serine protease polypeptide to the composition. Wash performance is compared under relevant washing conditions. In some test systems, other relevant factors, such as detergent composition, sud concentration, water hardness, washing mechanics, time, pH, and/or temperature, can be controlled in such a way that condition(s) typical for household application in a certain market segment (e.g., hand or manual dishwashing, automatic dishwashing, dishware cleaning, tableware cleaning, fabric cleaning, etc.) are imitated.

The term "relevant washing conditions" is used herein to indicate the conditions, particularly washing temperature, time, washing mechanics, sud concentration, type of detergent and water hardness, actually used in households in a hand dishwashing, automatic dishwashing, or laundry detergent market segment.

As used herein, the term "disinfecting" refers to the removal of contaminants from the surfaces, as well as the inhibition or killing of microbes on the surfaces of items. It is not intended that the present disclosure be limited to any particular surface, item, or contaminant(s) or microbes to be removed.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, the filler salt is sodium sulfate.

The present disclosure provides novel serine protease enzymes as defined in the claims. The serine protease polypeptides of the present disclosure include isolated, recombinant, substantially pure, or non- naturally occurring polypeptides. In some embodiments, the polypeptides are useful in cleaning applications and can be incorporated into cleaning compositions that are useful in methods of cleaning an item or a surface in need thereof.

In a first aspect the invention provides a subtilisin or recombinant polypeptide, or active fragment thereof, wherein said subtilisin, polypeptide or fragment comprises a XQXVPYGXEXIGAXDVQQND (SEQ ID NO:32) motif, wherein X is any amino acid, and wherein said subtilisin, polypeptide or fragment comprises an amino acid sequence having at least 85% amino acid sequence identity to the amino acid sequence SEQ ID NO: 3 or 6, and wherein said subtilisin, polypeptide or fragment has protease activity.

As noted above, the variant enzyme polypeptides of the invention as defined in the claims have enzymatic activities (e.g., protease activities) and thus are useful in cleaning applications, including but not limited to, methods for cleaning dishware items, tableware items, fabrics, and items having hard surfaces (e.g., the hard surface of a table, table top, wall, furniture item, floor, ceiling, etc.). Exemplary cleaning compositions comprising one or more variant serine protease enzyme polypeptides of the invention are described infra. The enzymatic activity (e.g., protease enzyme activity) of an enzyme polypeptide of the invention can be determined readily using procedures well known to those of ordinary skill in the art. The Examples presented infra describe methods for evaluating the enzymatic activity and cleaning performance. The performance of polypeptide enzymes of the invention in removing stains (e.g., a protein stain such as blood/milk/ink or egg yolk), cleaning hard surfaces, or cleaning laundry, dishware or tableware item(s) can be readily determined using procedures well known in the art and/or by using procedures set forth in the Examples.

The serine protease polypeptides of the present invention, as defined in the claims, can have protease activity over a broad range of pH conditions. In some embodiments, the serine protease polypeptides have protease activity on dimethylcasein as a substrate, as demonstrated in Examples below.

In some embodiments, the serine protease polypeptides of the present invention, as defined in the claims, demonstrate cleaning performance in a cleaning composition. Cleaning compositions often include ingredients harmful to the stability and performance of enzymes, making cleaning compositions a harsh environment for enzymes, e.g. serine proteases, to retain function. Thus, it is not trivial for an enzyme to be put in a cleaning composition and expect enzymatic function (e.g. serine protease activity, such as demonstrated by cleaning performance). In some embodiments, the serine protease polypeptides of the present invention demonstrate cleaning performance in automatic dishwashing (ADW) detergent compositions. In some embodiments, the cleaning performance in ADW detergent compositions includes cleaning of egg yolk stains. In some embodiments, the serine protease polypeptides of the present invention demonstrate cleaning performance in laundry detergent compositions. In some embodiments, the cleaning performance in laundry detergent compositions includes cleaning of blood/milk/ink stains. In each of the cleaning compositions, the serine protease polypeptides of the present invention demonstrate cleaning performance with or without a bleach component.

A polypeptide of the invention can be subject to various changes, such as one or moreamino acid insertions, deletions, and/or substitutions, either conservative or non-conservative, including where such changes do not substantially alter the enzymatic activity of the polypeptide, provided that said polypeptide is as defined in the claims. Similarly, a nucleic acid of the invention can also be subject to various changes, such as one or more substitutions of one or more nucleotides in one or more codons such that a particular codon encodes the same or a different amino acid, resulting in either a silent variation (e.g., when the encoded amino acid is not altered by the nucleotide mutation) or non-silent variation, one or more deletions of one or more nucleic acids (or codons) in the sequence, one or more additions or insertions of one or more nucleic acids (or codons) in the sequence, and/or cleavage of or one or more truncations of one or more nucleic acids (or codons) in the sequence. Many such changes in the nucleic acid sequence may not substantially alter the enzymatic activity of the resulting encoded polypeptide enzyme compared to the polypeptide enzyme encoded by the original nucleic acid sequence, provided that said polynucleotide is as defined in the claims. A nucleic acid sequence of the invention can also be modified to include one or more codons that provide for optimum expression in an expression system (e.g., bacterial expression system), while, if desired, said one or more codons still encode the same amino acid(s), provided that said nucleic acid sequence is as defined in the claims.

The invention provides isolated, non-naturally occurring, or recombinant nucleic acids which may be collectively referred to as "nucleic acids of the invention" or "polynucleotides of the invention", which encode polypeptides of the invention, as defined in the claims. Nucleic acids of the invention, including all described below, are useful in recombinant production (e.g., expression) of polypeptides of the invention, typically through expression of a plasmid expression vector comprising a sequence encoding the polypeptide of interest or fragment thereof. As discussed above, polypeptides include serine protease polypeptides having enzymatic activity (e.g., proteolytic activity) which are useful in cleaning applications and cleaning compositions for cleaning an item or a surface (e.g., surface of an item) in need of cleaning.

In some embodiments, the polynucleotide of the present invention is a polynucleotide having a specified degree of nucleic acid homology to the exemplified polynucleotide. In some embodiments, the polynucleotide comprises a nucleic acid sequence having at least 85, 90, 91, 92, 93, 94, 95, 96, 97, 98, 99 or 100% identity to the nucleic acid sequence of SEQ ID NO:1 or 4. In some embodiments, the polynucleotide comprises a nucleic acid sequence selected from the group consisting of SEQ ID NOs:1 and 4.

Nucleic acids of the invention can be generated by using any suitable synthesis, manipulation, and/or isolation techniques, or combinations thereof, provided that said nucleic acid is as defined in the claims. For example, a polynucleotide of the invention may be produced using standard nucleic acid synthesis techniques, such as solid-phase synthesis techniques that are well-known to those skilled in the art. In such techniques, fragments of up to 50 or more nucleotide bases are typically synthesized, then joined (e.g., by enzymatic or chemical ligation methods) to form essentially any desired continuous nucleic acid sequence. The synthesis of the nucleic acids of the invention can be also facilitated by any suitable method known in the art, including but not limited to chemical synthesis using the classical phosphoramidite method (See e.g., Beaucage et al. Tetrahedron Letters 22:1859-69 [1981]); or the method described by Matthes et al. (See, Matthes et al., EMBO J. 3:801-805 [1984], as is typically practiced in automated synthetic methods. Nucleic acids of the invention also can be produced by using an automatic DNA synthesizer. Customized nucleic acids can be ordered from a variety of commercial sources (e.g., The Midland Certified Reagent Company, the Great American Gene Company, Operon Technologies Inc., and DNA2.0). Other techniques for synthesizing nucleic acids and related principles are known in the art (See e.g., Itakura et al., Ann. Rev. Biochem. 53:323 [1984]; and Itakura et al., Science 198:1056 [1984]).

As indicated above, recombinant DNA techniques useful in modification of nucleic acids are well known in the art. For example, techniques such as restriction endonuclease digestion, ligation, reverse transcription and cDNA production, and polymerase chain reaction (e.g., PCR) are known and readily employed by those of skill in the art. Nucleotides of the invention may also be obtained by screening cDNA libraries using one or more oligonucleotide probes that can hybridize to or PCR-amplify polynucleotides which encode a serine protease polypeptide polypeptide(s) of the invention. Procedures for screening and isolating cDNA clones and PCR amplification procedures are well known to those of skill in the art and described in standard references known to those skilled in the art. Some nucleic acids of the invention can be obtained by altering a naturally occurring polynucleotide backbone (e.g., that encodes an enzyme or parent protease) by, for example, a known mutagenesis procedure (e.g., site-directed mutagenesis, site saturation mutagenesis, and in vitro recombination). A variety of methods are known in the art that are suitable for generating modified polynucleotides of the invention that encode serine protease polypeptides of the invention, including, but not limited to, for example, site-saturation mutagenesis, scanning mutagenesis, insertional mutagenesis, deletion mutagenesis, random mutagenesis, site-directed mutagenesis, and directed-evolution, as well as various other recombinatorial approaches.

The present invention provides vectors comprising at least one serine protease polynucleotide of the invention as defined in the claims, expression vectors or expression cassettes comprising at least one nucleic acid or polynucleotide of the invention, isolated, substantially pure, or recombinant DNA constructs comprising at least one nucleic acid or polynucleotide of the invention, isolated or recombinant cells comprising at least one polynucleotide of the invention, and compositions comprising one or more such vectors, nucleic acids, expression vectors, expression cassettes, DNA constructs, cells, cell cultures, or any combination or mixtures thereof.

In some embodiments, the invention provides recombinant cells comprising at least one vector (e.g., expression vector or DNA construct) of the invention, as defined in the claims, which comprises at least one nucleic acid or polynucleotide of the invention as defined in the claims. Some such recombinant cells are transformed or transfected with such at least one vector, although other methods are available and known in the art. Such cells are typically referred to as host cells. Some such cells comprise bacterial cells, including, but are not limited to *Bacillus sp.* cells, such as *B. subtilis* cells. The invention also provides recombinant cells (e.g., recombinant host cells) comprising at least one serine protease polypeptide of the invention as defined in the claims.

In some embodiments, the invention provides a vector comprising a nucleic acid or polynucleotide of the invention as defined in the claims. In some embodiments, the vector is an expression vector or expression cassette in which a polynucleotide sequence of the invention which encodes a serine protease polypeptide of the invention, provided that said polypeptide is as defined in the claims, is operably linked to one or additional nucleic acid segments required for efficient gene expression (e.g., a promoter operably linked to the polynucleotide of the invention which encodes a serine protease polypeptide of the invention). A vector may include a transcription terminator and/or a selection gene, such as an antibiotic resistance gene, that enables continuous cultural maintenance of plasmid-infected host cells by growth in antimicrobial-containing media.

For expression and production of a serine protease polypeptide as defined in the claims in a cell, at least one expression vector comprising at least one copy of a Polynucleotide, as defined in the claims, encoding the serine protease polypeptide, and in some instances comprising multiple copies, is transformed into the cell under conditions suitable for expression of the serine protease. In some embodiments of the present invention, a polynucleotide sequence encoding the serine protease polypeptide as defined in the claims is integrated into the genome of the host cell, while in other embodiments, a plasmid vector comprising a polynucleotide sequence encoding the serine protease polypeptide remains as autonomous extra- chromosomal element within the cell. The invention provides both extrachromosomal nucleic acid elements as well as incoming nucleotide sequences that are integrated into the host cell genome. The vectors described herein are useful for production of the serine protease polypeptides of the invention. In some embodiments, a polynucleotide construct encoding the serine protease polypeptide is present on an integrating vector that enables the integration and optionally the amplification of the polynucleotide encoding the serine protease polypeptide into the host chromosome. Examples of sites for integration are well known to those skilled in the art. In some embodiments, transcription of a polynucleotide encoding a serine protease polypeptide of the invention is effectuated by a promoter that is the wild-type promoter for the selected precursor protease. In some other embodiments, the promoter is heterologous to the precursor protease, but is functional in the host cell. Specifically, examples of suitable promoters for use in bacterial host cells include, but are not limited to, for example, the amyE, amyQ, amyL, pstS, sacB, pSPAC, pAprE, pVeg, pHpaII promoters, the promoter of the *B. stearothermophilus* maltogenic amylase gene, the *B. amyloliquefaciens* (BAN) amylase gene, the *B. subtilis* alkaline protease gene, the *B. clausii* alkaline protease gene the *B. pumilis* xylosidase gene, the *B. thuringiensis* cryIIIA, and the *B. licheniformis* alpha-amylase gene. Additional promoters include, but are not limited to the A4 promoter, as well as phage Lambda PR or PL promoters, and the *E. coli* lac, trp or tac promoters.

Serine protease polypeptides of the present invention, as defined in the claims, can be produced in host cells of any suitable microorganism, including bacteria and fungi. In some embodiments, serine protease polypeptides of the present invention can be produced in Gram-positive bacteria. In some embodiments, the host cells are *Bacillus spp., Streptomyces spp., Escherichia spp., Aspergillus spp., Trichoderma spp., Pseudomonas spp., Corynebacterium spp., Saccharomyces spp.,* or *Pichia spp.* In some embodiments, the serine protease polypeptides are produced by *Bacillus sp.* host cells. Examples of *Bacillus sp.* host cells that find use in the production of the serine protease polypeptides of the invention include, but are not limited to *B. licheniformis, B. lentus, B. subtilis, B. amyloliquefaciens, B. lentus, B. brevis, B. stearothermophilus, B. alkalophilus, B. coagulans, B. circulans, B. pumilis, B. thuringiensis, B. clausii,* and *B. megaterium,* as well as other organisms within the genus *Bacillus.* In some embodiments, *B. subtilis* host cells are used for production of serine protease polypeptides. US 5,264,366 and 4,760,025 (RE 34,606) describe various *Bacillus* host strains that can be used for producing serine protease polypeptide of the invention, although other suitable strains can be used.

Several bacterial strains that can be used to produce serine protease polypeptides of the invention, as defined in the claims, include non-recombinant (i.e., wild-type) *Bacillus sp.* strains, as well as variants of naturally-occurring strains and/or recombinant strains. In some embodiments, the host strain is a recombinant strain, wherein a polynucleotide encoding a polypeptide of interest has been introduced into the host. In some embodiments, the host strain is a *B. subtilis* host strain and particularly a recombinant *B. subtilis* host strain. Numerous *B. subtilis* strains are known, including, but not limited to for example, 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain (See e.g., Hoch et al., Genetics 73:215-228 [1973]; See also, US 4,450,235 and 4,302,544, and EP 0134048). The use of *B. subtilis* as an expression host cells is well known in the art (See e.g., Palva et al., Gene 19:81-87 [1982]; Fahnestock and Fischer, J. Bacteriol., 165:796-804 [1986]; and Wang et al., Gene 69:39-47 [1988]).

Host cells are transformed with at least one nucleic acid encoding at least one serine protease polypeptide of the invention, as defined in the claims, using any suitable method known in the art. Methods for introducing a nucleic acid (e.g., DNA) into *Bacillus* cells or *E. coli* cells utilizing plasmid DNA constructs or vectors and transforming such plasmid DNA constructs or vectors into such cells are well known. In some embodiments, the plasmids are subsequently isolated from *E. coli* cells and transformed into *Bacillus* cells. However, it is not essential to use intervening microorganisms such as *E. coli,* and in some embodiments, a DNA construct or vector is directly introduced into a *Bacillus* host.

Those of skill in the art are well aware of suitable methods for introducing nucleic acid sequences of the invention, as defined in the claims, into *Bacillus* cells (See e.g., Ferrari et al., "Genetics," in Harwood et al. [eds.], Bacillus, Plenum Publishing Corp. [1989], pp. 57-72; Saunders et al., J. Bacteriol. 157:718-726 [1984]; Hoch et al., J. Bacteriol. 93:1925 -1937 [1967]; Mann et al., Current Microbiol. 13:131-135 [1986]; Holubova, Folia Microbiol. 30:97 [1985]; Chang et al., Mol. Gen. Genet. 168:11-115 [1979]; Vorobjeva et al., FEMS Microbiol. Lett. 7:261-263 [1980]; Smith et al., Appl. Env. Microbiol. 51:634 [1986]; Fisher et al., Arch. Microbiol. 139:213-217 [1981]; and McDonald, J. Gen. Microbiol. 130:203 [1984]). Indeed, such methods as transformation, including protoplast transformation and transfection, transduction, and protoplast fusion are well known and suited for use in the present invention. Methods known in the art to transform *Bacillus* cells include such methods as plasmid marker rescue transformation, which involves the uptake of a donor plasmid by competent cells carrying a partially homologous resident plasmid (See, Contente et al., Plasmid 2:555-571 [1979]; Haima et al., Mol. Gen. Genet. 223:185-191 [1990]; Weinrauch et al., J. Bacteriol. 154:1077-1087 [1983]; and Weinrauch et al., J. Bacteriol. 169:1205-1211 [1987]). In this method, the incoming donor plasmid recombines with the homologous region of the resident "helper" plasmid in a process that mimics chromosomal transformation.

In addition to commonly used methods, in some embodiments, host cells are directly transformed with a DNA construct or vector comprising a nucleic acid encoding a serine protease polypeptide of the invention as defined in the claims (i.e., an intermediate cell is not used to amplify, or otherwise process, the DNA construct or vector prior to introduction into the host cell). Introduction of the DNA construct or vector of the invention into the host cell includes those physical and chemical methods known in the art to introduce a nucleic acid sequence (e.g., DNA sequence) into a host cell without insertion into the host genome. Such methods include, but are not limited to calcium chloride precipitation, electroporation, naked DNA, liposomes and the like. In additional embodiments, DNA constructs or vector are co-transformed with a plasmid, without being inserted into the plasmid. In further embodiments, a selective marker is deleted from the altered Bacillus strain by methods known in the art (See, Stahl et al., J. Bacteriol. 158:411-418 [1984]; and Palmeros et al., Gene 247:255 -264 [2000]).

In some other embodiments, the invention provides methods for making or producing a mature serine protease polypeptide of the invention, as defined in the claims. A mature serine protease polypeptide does not include a signal peptide or a propeptide sequence. Some methods, as defined in the claims, comprise making or producing a serine protease polypeptide of the invention in a recombinant bacterial host cell, such as for example, a *Bacillus* sp. cell (e.g., a *B. subtilis* cell). In some embodiments, the invention provides a method of producing a serine protease polypeptide of the invention, the method comprising cultivating a recombinant host cell comprising a recombinant expression vector comprising a nucleic acid encoding a serine protease polypeptide of the invention under conditions conducive to the production of the serine protease polypeptide, provided that said polypeptide is as defined in the claims. Some such methods further comprise recovering the serine protease polypeptide from the culture.

In some embodiments the invention provides methods as defined in the claims of producing a serine protease polypeptide of the invention, the methods comprising: (a) introducing a recombinant expression vector comprising a nucleic acid encoding a serine protease polypeptide of the invention into a population of cells; and (b) culturing the cells in a culture medium under conditions conducive to produce the serine protease polypeptide encoded by the expression vector. Some such methods further comprise: (c) isolating the serine protease polypeptide from the cells or from the culture medium.

Unless otherwise noted, all component or composition levels provided herein are made in reference to the active level of that component or composition, and are exclusive of impurities, for example, residual solvents or by-products, which maybe present in commercially available sources. Enzyme components weights are based on total active protein. All percentages and ratios are calculated by weight unless otherwise indicated. All percentages and ratios are calculated based on the total composition unless otherwise indicated. Compositions of the invention include cleaning compositions, such as detergent compositions. In the exemplified detergent compositions, the enzymes levels are expressed by pure enzyme by weight of the total composition and unless otherwise specified, the detergent ingredients are expressed by weight of the total compositions.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the serine protease polypeptides of the present invention, provided that said polypeptide is as defined in the claims. The precise nature of these additional components, and levels of incorporation thereof, will depend on the physical form of the composition and the nature of the cleaning operation for which it is to be used. Suitable adjunct materials include, but are not limited to, bleach catalysts, other enzymes, enzyme stabilizing systems, chelants, optical brighteners, soil release polymers, dye transfer agents, dispersants, suds suppressors, dyes, perfumes, colorants, filler salts, , photoactivators, fluorescers, fabric conditioners, hydrolyzable surfactants, preservatives, anti- oxidants, anti-shrinkage agents, anti-wrinkle agents, germicides, fungicides, color speckles, silvercare, anti-tarnish and/or anticorrosion agents, alkalinity sources, solubilizing agents, carriers, processing aids, pigments, and pH control agents, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti- redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in US 5,576,282; 6,306,812; 6,326,348; 6,610,642; 6,605,458; 5,705,464; 5,710,115; 5,698,504;
5,695,679; 5,686,014; and 5,646,101. In embodiments in which the cleaning adjunct materials are not compatible with the serine protease polypeptides of the present invention in the cleaning compositions, then suitable methods of keeping the cleaning adjunct materials and the protease(s) separated (i.e., not in contact with each other) until combination of the two components is appropriate are used. Such separation methods include any suitable method known in the art (e.g., gelcaps, encapsulation, tablets, physical separation, etc.). The aforementioned adjunct ingredients may constitute the balance of the cleaning compositions of the present invention.

The cleaning compositions of the present invention as defined in the claims are advantageously employed for example, in laundry applications, hard surface cleaning applications, dishwashing applications, including automatic dishwashing and hand dishwashing, as well as cosmetic applications such as dentures, teeth, hair and skin cleaning. The enzymes of the present invention as defined in the claims are also suited for use in contact lens cleaning and wound debridement applications. In addition, due to the unique advantages of increased effectiveness in lower temperature solutions, the enzymes of the present invention are ideally suited for laundry applications. Furthermore, the enzymes of the present invention find use in granular and liquid compositions.

The serine protease polypeptides of the present invention, provided that said polypeptides are as defined in the claims also find use in cleaning additive products. In some embodiments, low temperature solution cleaning applications find use. In some embodiments, the present invention provides cleaning additive products including at least one enzyme of the present invention is ideally suited for inclusion in a wash process when additional bleaching effectiveness is desired. Such instances include, but are not limited to low temperature solution cleaning applications. In some embodiments, the additive product is in its simplest form, one or more proteases. In some embodiments, the additive is packaged in dosage form for addition to a cleaning process. In some embodiments, the additive is packaged in dosage form for addition to a cleaning process where a source of peroxygen is employed and increased bleaching effectiveness is desired. Any suitable single dosage unit form finds use with the present invention, including but not limited to pills, tablets, gelcaps, or other single dosage units such as pre-measured powders or liquids. In some embodiments, filler(s) or carrier material(s) are included to increase the volume of such compositions. Suitable filler or carrier materials include, but are not limited to, various salts of sulfate, carbonate and silicate as well as talc, clay and the like. Suitable filler or carrier materials for liquid compositions include, but are not limited to water or low molecular weight primary and secondary alcohols including polyols and diols. Examples of such alcohols include, but are not limited to, methanol, ethanol, propanol and isopropanol. In some embodiments, the compositions contain from about 5 to about 90% of such materials. Acidic fillers find use to reduce pH. Alternatively, in some embodiments, the cleaning additive includes adjunct ingredients, as more fully described below.

The present cleaning compositions and cleaning additives require an effective amount of at least one of the serine protease polypeptides as defined in the claims, alone or in combination with other proteases and/or additional enzymes. The required level of enzyme is achieved by the addition of one or more serine protease polypeptides of the present invention. Typically the present cleaning compositions comprise at least about 0.0001 weight percent, from about 0.0001 to about 10, from about 0.001 to about 1, or from about 0.01 to about 0.1 weight percent of at least one of the serine protease polypeptides of the present invention.

The cleaning compositions as defined in the claims are typically formulated such that, during use in aqueous cleaning operations, the wash water will have a pH of from about 4.0 to about 11.5, or even from about 5.0 to about 11.5, or even from about 5.0 to about 8.0, or even from about 7.5 to about 10.5. Liquid product formulations are typically formulated to have a pH from about 3.0 to about 9.0 or even from about 3 to about 5. Granular laundry products are typically formulated to have a pH from about 9 to about 11. In some embodiments, the cleaning compositions of the present invention, as defined in the claims, can be formulated to have an alkaline pH under wash conditions, such as a pH of from about 8.0 to about 12.0, or from about 8.5 to about 11.0, or from about 9.0 to about 11.0. In some embodiments, the cleaning compositions of the present invention can be formulated to have a neutral pH under wash conditions, such as a pH of from about 5.0 to about 8.0, or from about 5.5 to about 8.0, or from about 6.0 to about 8.0, or from about 6.0 to about 7.5. In some embodiments, the neutral pH conditions can be measured when the cleaning composition is dissolved 1:100 (wt:wt) in deionised water at 20°C, measured using a conventional pH meter. Techniques for controlling pH at recommended usage levels include the use of buffers, alkalis, acids, etc., and are well known to those skilled in the art.

In some embodiments, when the serine protease polypeptide(s) as defined in the claims is/are employed in a granular composition or liquid, it is desirable for the serine protease polypeptide to be in the form of an encapsulated particle to protect the serine protease polypeptide from other components of the granular composition during storage. In addition, encapsulation is also a means of controlling the availability of the serine protease polypeptide during the cleaning process. In some embodiments, encapsulation enhances the performance of the serine protease polypeptide(s) and/or additional enzymes. In this regard, the serine protease polypeptide(s) of the present invention are encapsulated with any suitable encapsulating material known in the art. In some embodiments, the encapsulating material typically encapsulates at least part of the serine protease polypeptide(s) of the present invention. Typically, the encapsulating material is water- soluble and/or water-dispersible. In some embodiments, the encapsulating material has a glass transition temperature (Tg) of 0°C or higher. Glass transition temperature is described in more detail in WO97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch (See e.g., EP0922499; and US 4,977,252; 5,354,559; and 5,935,826). In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include, but are not limited to those supplied by EXPANCEL® (Stockviksverken, Sweden), and PM6545, PM6550, PM7220, PM7228, EXTENDOSPHERES®, LUXSIL®, Q-CEL®, and SPHERICEL® (PQ Corp., Valley Forge, PA).

There are a variety of wash conditions including varying detergent formulations, wash water volumes, wash water temperatures, and lengths of wash time, to which proteases involved in washing are exposed. A low detergent concentration system includes detergents where less than about 800 ppm of the detergent components is present in the wash water. A medium detergent concentration includes detergents where between about 800 and about 2000 ppm of the detergent components is present in the wash water. A high detergent concentration system includes detergents where greater than about 2000 ppm of the detergent components is present in the wash water. In some embodiments, the "cold water washing" of the present invention utilizes "cold water detergent" suitable for washing at temperatures from about 10°C to about 40°C, or from about 20°C to about 30°C, or from about 15°C to about 25°C, as well as all other combinations within the range of about 15°C to about 35°C, and all ranges within 10°C to 40°C.

Different geographies typically have different water hardness. Water hardness is usually described in terms of the grains per gallon mixed Ca²⁺/Mg²⁺. Hardness is a measure of the amount of calcium (Ca²⁺) and magnesium (Mg²⁺) in the water. Most water in the United States is hard, but the degree of hardness varies. Moderately hard (60-120 ppm) to hard (121- 181 ppm) water has 60 to 181 parts per million.

**Table I. Water Hardness**

| Water | Grains per gallon | Parts per million |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

In some embodiments of the present invention, the cleaning compositions comprise at least one serine protease polypeptide of the present invention, as defined in the claims, at a level from about 0.00001 to about 10% by weight of the composition and the balance (e.g., about 99.999 to about 90.0%) comprising cleaning adjunct materials by weight of composition. In some other embodiments of the present invention, the cleaning compositions of the present invention comprises at least one serine protease polypeptide at a level of about 0.0001 to about 10%, about 0.001 to about 5%, about 0.001 to about 2%, about 0.005 to about 0.5% by weight of the composition and the balance of the cleaning composition (e.g., about 99.9999 to about 90.0%, about 99.999 to about 98%, about 99.995 to about 99.5% by weight) comprising cleaning adjunct materials.

In some embodiments, the cleaning compositions of the present invention, as defined in the claims, comprise one or more additional detergent enzymes, which provide cleaning performance and/or fabric care and/or dishwashing benefits. Examples of suitable enzymes include, but are not limited to, acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo- mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta- glucanases, tannases, transglutaminases, xylan acetyl-esterases, xylanases, xyloglucanases, and xylosidases, or any combinations or mixtures thereof. In some embodiments, a combination of enzymes is used (i.e., a "cocktail") comprising conventional applicable enzymes like protease, lipase, cutinase and/or cellulase in conjunction with amylase is used.

In addition to the serine protease polypeptides provided as defined in the claims, any other suitable protease finds use in the compositions of the present invention. Suitable proteases include those of animal, vegetable or microbial origin. In some embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from *Bacillus* (e.g., subtilisin, *lentus, amyloliquefaciens,* subtilisin Carlsberg, subtilisin 309, subtilisin 147 and subtilisin 168). Additional examples include those mutant proteases described in U.S. Pat. Nos. RE 34,606, 5,955,340, 5,700,676, 6,312,936, and 6,482,628. Additional protease examples include, but are not limited to trypsin (e.g., of porcine or bovine origin), and the *Fusarium* protease described in WO89/06270. In some embodiments, commercially available protease enzymes that find use in the present invention include, but are not limited to MAXATASE®, MAXACAL™, MAXAPEM™, OPTICLEAN®, OPTIMASE®, PROPERASE®, PURAFECT®, PURAFECT® OXP, PURAMAX™, EXCELLASE™, PREFERENZ™ proteases (e.g. P100, P110, P280), EFFECTENZ™ proteases (e.g. P1000, P1050, P2000), EXCELLENZ™ proteases (e.g. P1000), ULTIMASE®, and PURAFAST™ (Genencor); ALCALASE®, SAVINASE®, PRIMASE®, DURAZYM™, POLARZYME®, OVOZYME®, KANNASE®, LIQUANASE®, NEUTRASE®, RELASE® and ESPERASE® (Novozymes); BLAP™ and BLAP™ variants (Henkel Kommanditgesellschaft aufAktien, Duesseldorf, Germany), and KAP (*B. alkalophilus* subtilisin; Kao Corp., Tokyo, Japan). Various proteases are described in WO95/23221, WO92/21760, WO 09/149200, WO09/149144, WO 9/149145, WO11/072099, WO10/056640, WO10/056653, WO 11/140364, WO12/151534, US 2008/0090747, and US 5,801,039; 5,340,735; 5,500,364; 5,855,625; RE 34,606; 5,955,340; 5,700,676; 6,312,936; 6,482,628; and 8,530,219; and various other patents. In some further embodiments, neutral metalloproteases find use in the present invention, including but not limited to the neutral metalloproteases described in WO1999014341, WO1999033960, WO1999014342, WO1999034003, WO2007044993, WO2009058303, WO 2009058661, WO2014/071410, WO2014/194032, WO2014/194034, WO2014/194054, and WO2014/194117. Exemplary metalloproteases include nprE, the recombinant form of neutral metalloprotease expressed in *B. subtilis* (See e.g., WO07/044993), and PMN, the purified neutral metalloprotease from *B amyloliquefaciens.*

In addition, any suitable lipase finds use in compositions of the present invention as defined in the claims. Suitable lipases include, but are not limited to those of bacterial or fungal origin. Chemically or genetically modified mutants are encompassed by the present invention. Examples ofuseful lipases include *H. lanuginosa* lipase (See e.g., EP258068, and EP305216), *Rhizomucor miehei* lipase (See e.g., EP238023), *Candida lipase,* such as *C*. *antarctica* lipase (e.g., *C*. *antarctica* lipase A or B; See e.g., EP214761), *Pseudomonas* lipases such as *P. alcaligenes* lipase and *P. pseudoalcaligenes* lipase (See e.g., EP218272), *P. cepacia* lipase (See e.g., EP331376), *P. stutzeri* lipase (See e.g., GB 1,372,034), *P.fluorescens* lipase, *Bacillus* lipase (e.g., *B. subtilis* lipase [Dartois et al., Biochem. Biophys. Acta 1131:253-260 [1993]); *B. stearothermophilus* lipase [See e.g., JP64/744992]; and *B. pumilus* lipase [See e.g., WO91/16422]).

Furthermore, a number of cloned lipases find use in some compositions of the present invention, as defined in the claims, including but not limited to *Penicillium camembertii* lipase (See, Yamaguchi et al., Gene 103:61-67 [1991]), *Geotricum candidum* lipase (See, Schimada et al., J. Biochem., 106:383-388 [1989]), and various *Rhizopus* lipases such as *R. delemar* lipase (See, Hass et al., Gene 109:117-113 [1991]), a *R. niveus* lipase (Kugimiya et al., Biosci. Biotech. Biochem. 56:716-719 [1992]) and *R*. *oryzae* lipase.

Other types of lipase polypeptide enzymes such as cutinases also find use in some compositions of the present invention, as defined in the claims, including but not limited to the cutinase derived from *Pseudomonas mendocina* (See, WO88/09367), and the cutinase derived from *Fusarium solani pisi* (See, WO 90/09446).

Additional suitable lipases include lipases such as M1 LIPASE™, LUMA FAST™, and LIPOMAX™ (Genencor); LIPEX®, LIPOLASE® and LIPOLASE® ULTRA (Novozymes); and LIPASEP™ "Amano" (Amano Pharmaceutical Co. Ltd., Japan).

In some embodiments of the present invention, the cleaning compositions of the present invention, as defined in the claims, further comprise lipases at a level from about 0.00001 to about 10% of additional lipase by weight of the composition and the balance of cleaning adjunct materials by weight of composition. In some other embodiments of the present invention, the cleaning compositions of the present invention also comprise lipases at a level of about 0.0001 to about 10%, about 0.001 to about 5%, about 0.001 to about 2%, about 0.005 to about 0.5% lipase by weight of the composition.

In some compositions of the present invention, as defined in the claims, any suitable amylase finds use in the present invention. In some embodiments, any amylase (e.g., alpha and/or beta) suitable for use in alkaline solutions also find use. Suitable amylases include, but are not limited to those of bacterial or fungal origin. Chemically or genetically modified mutants are included in some embodiments. Amylases that find use in the present invention, include, but are not limited to α- amylases obtained from *B. licheniformis* (See e.g., GB 1,296,839). Additional suitable amylases include those found in WO9510603, WO9526397, WO9623874, WO9623873, WO9741213, WO 9919467, WO0060060, WO0029560, WO9923211, WO9946399, WO0060058, WO0060059, WO9942567, WOOl 14532, WO02092797, WO0166712, WO0188107, WO0196537, WO 0210355, WO9402597, WO0231124, WO9943793, WO9943794, WO2004113551, WO2005001064, WO2005003311, WO0164852, WO2006063594, WO2006066594, WO2006066596, WO2006012899, WO2008092919, WO2008000825, WO2005018336, WO2005066338, WO2009140504, WO2005019443, WO2010091221, WO2010088447, WO0134784, WO2006012902, WO2006031554, WO2006136161, WO2008101894, WO2010059413, WO2011098531, WO2011080352, WO2011080353, WO2011080354, WO2011082425, WO2011082429, WO2011076123, WO2011087836, WO2011076897, WO94183314, WO9535382, WO9909183, WO9826078, WO9902702, WO9743424, WO9929876, WO9100353, WO9605295, WO9630481, WO9710342, WO2008088493, WO2009149419, WO 2009061381, WO2009100102, WO2010104675, WO2010117511, and WO2010115021.
Commercially available amylases that find use in the present invention include, but are not limited to DURAMYL®, TERMAMYL®, FUNGAMYL®, STAINZYME®, STAINZYME PLUS®, STAINZYME ULTRA®, and BAN™ (Novozymes), as well as POWERASE™, RAPIDASE® and MAXAMYL® P (Genencor).

In some embodiments of the present invention, the cleaning compositions of the present invention, as defined in the claims, further comprise amylases at a level from about 0.00001 to about 10% of additional amylase by weight of the composition and the balance of cleaning adjunct materials by weight of composition. In some other embodiments of the present invention, the cleaning compositions of the present invention also comprise amylases at a level of about 0.0001 to about 10%, about 0.001 to about 5%, about 0.001 to about 2%, about 0.005 to about 0.5% amylase by weight of the composition.

In some further embodiments, any suitable cellulase finds used in the cleaning compositions of the present invention, as defined in the claims. Suitable cellulases include, but are not limited to those of bacterial or fungal origin. Chemically or genetically modified mutants are included in some embodiments. Suitable cellulases include, but are not limited to *Humicola insolens* cellulases (See e.g., US 4,435,307). Especially suitable cellulases are the cellulases having color care benefits (See e.g., EP 0 495 257). Commercially available cellulases that find use in the present include, but are not limited to CELLUZYME®, CAREZYME® (Novozymes), REVITALENZ™
100 (Danisco US Inc) and KAC-500(B)™ (Kao Corporation). In some embodiments, cellulases are incorporated as portions or fragments of mature wild-type or variant cellulases, wherein a portion of the N-terminus is deleted (See e.g., US 5,874,276). Additional suitable cellulases include those found in WO2005054475, WO2005056787, US 7,449,318 and US 7,833,773. In some embodiments, the cleaning compositions of the present invention further comprise cellulases at a level from about 0.00001 to about 10% of additional cellulase by weight of the composition and the balance of cleaning adjunct materials by weight of composition. In some other embodiments of the present invention, the cleaning compositions of the present invention also comprise cellulases at a level of about 0.0001 to about 10%, about 0.001 to about 5%, about 0.001 to about 2%, about 0.005 to about 0.5% cellulase by weight of the composition.

Any mannanase suitable for use in detergent compositions also finds use in the present invention, provided the compositions are defined in the claims. Suitable mannanases include, but are not limited to those of bacterial or fungal origin. Chemically or genetically modified mutants are included in some embodiments. Various mannanases are known which find use in the present invention (See e.g., US 6,566,114; 6,602,842; and 6,440,991). Commercially available mannanases that find use in the present invention include, but are not limited to MANNASTAR®, PURABRITE™, and MANNAWAY®. In some embodiments, the cleaning compositions of the present invention further comprise mannanases at a level from about 0.00001 to about 10% of additional mannanase by weight of the composition and the balance of cleaning adjunct materials by weight of composition. In some embodiments of the present invention, the cleaning compositions of the present invention also comprise mannanases at a level of about 0.0001 to about 10%, about 0.001 to about 5%, about 0.001 to about 2%, about 0.005 to about 0.5% mannanase by weight of the composition.

In some embodiments, peroxidases are used in combination with hydrogen peroxide or a source thereof (e.g., a percarbonate, perborate or persulfate) in the compositions of the present invention, provided said compositions are defined in the claims. In some alternative embodiments, oxidases are used in combination with oxygen. Both types of enzymes are used for "solution bleaching" (i.e., to prevent transfer of a textile dye from a dyed fabric to another fabric when the fabrics are washed together in a wash liquor), preferably together with an enhancing agent (See e.g., WO94/12621 and WO95/01426). Suitable peroxidases/oxidases include, but are not limited to those of plant, bacterial or fungal origin. Chemically or genetically modified mutants are included in some embodiments. In some embodiments, the cleaning compositions of the present invention further comprise peroxidase and/or oxidase enzymes at a level from about 0.00001 % to about 10% of additional peroxidase and/or oxidase by weight of the composition and the balance of cleaning adjunct materials by weight of composition. In some other embodiments of the present invention, the cleaning compositions of the present invention also comprise peroxidase and/or oxidase enzymes at a level of about 0.0001 to about 10%, about 0.001 to about 5%, about 0.001 to about 2%, about 0.005 to about 0.5% peroxidase and/or oxidase enzymes by weight of the composition.

In some embodiments of compositions provided in the claims, additional enzymes find use, including but not limited to perhydrolases (See e.g., WO2005056782, WO2007106293, WO2008063400, WO2008106214, and WO2008106215). In addition, in some embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the serine protease polypeptide (s) and one or more additional enzymes may both independently range to about 10%, the balance of the cleaning composition being cleaning adjunct materials. The specific selection of cleaning adjunct materials are readily made by considering the surface, item, or fabric to be cleaned, and the desired form of the composition for the cleaning conditions during use (e.g., through the wash detergent use).

In some embodiments, an effective amount of one or more serine protease polypeptide (s) provided in the claims is included in compositions useful for cleaning a variety of surfaces in need ofproteinaceous stain removal. Such cleaning compositions include cleaning compositions for such applications as cleaning hard surfaces, fabrics, and dishes. Indeed, in some embodiments, the present invention provides fabric cleaning compositions, while in other embodiments the present invention provides non-fabric cleaning compositions. Notably, the present invention also provides cleaning compositions suitable for personal care, including oral care (including dentrifices, toothpastes, mouthwashes, etc., as well as denture cleaning compositions), skin, and hair cleaning compositions. It is intended that the present invention encompass detergent compositions in any form (i.e., liquid, granular, bar, semi-solid, gels, emulsions, tablets, capsules, etc.).

Several cleaning compositions wherein the serine protease polypeptides of the present invention provided in the claims find use are described in greater detail below. In some embodiments in which the cleaning compositions of the present invention are formulated as compositions suitable for use in laundry machine washing method(s), the compositions of the present invention contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (i.e., as additional cleaning adjunct materials). The compositions of the present invention also find use in detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments it ranges from about 500 to about 950 g/liter of composition measured at 20°C.

In embodiments formulated as compositions for use in manual dishwashing methods, the compositions of the invention, as defined in the claims, contain at least one surfactant and preferably at least one additional cleaning adjunct material selected from organic polymeric compounds, suds enhancing agents, group II metal ions, solvents, hydrotropes and additional enzymes.

In some embodiments, various cleaning compositions such as those provided in US 6,605,458 find use with the serine protease polypeptides of the present invention, as defined in the claims. Thus, in some embodiments, the compositions comprising at least one serine protease polypeptide of the present invention is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions comprising at least one serine protease polypeptide of the present invention are fabric cleaning compositions such as those described in US 6,610,642 and 6,376,450. In addition, the serine protease polypeptides of the present invention find use in granular laundry detergent compositions of particular utility under European or Japanese washing conditions (See e.g., US 6,610,642).

In some alternative embodiments, the present invention provides hard surface cleaning compositions comprising at least one serine protease polypeptide provided in the claims. Thus, in some embodiments, the compositions comprising at least one serine protease polypeptide of the present invention is a hard surface cleaning composition such as those described in US 6,610,642; 6,376,450; and 6,376,450.

In yet further embodiments, the present invention provides dishwashing compositions comprising at least one serine protease polypeptide provided as defined in the claims. Thus, in some embodiments, the compositions comprising at least one serine protease polypeptide of the present invention is a hard surface cleaning composition such as those in US 6,610,642 and 6,376,450. In some still further embodiments, the present invention provides dishwashing compositions comprising at least one serine protease polypeptide provided herein. In some further embodiments, the compositions comprising at least one serine protease polypeptide of the present invention comprise oral care compositions such as those in US 6,376,450, and 6,376,450. The formulations and descriptions of the compounds and cleaning adjunct materials contained in the aforementioned US 6,376,450; 6,605,458; 6,605,458; and 6,610,642 find use with the serine protease polypeptides provided herein.

The cleaning compositions of the present invention, provided as defined in the claims, are formulated into any suitable form and prepared by any process chosen by the formulator, non-limiting examples of which are described in US 5,879,584; 5,691,297; 5,574,005; 5,569,645; 5,565,422; 5,516,448; 5,489,392; and 5,486,303. When a low pH cleaning composition is desired, the pH of such composition is adjusted via the addition of a material such as monoethanolamine or an acidic material such as HCl.

In some embodiments, the cleaning compositions according to the present invention, as defined in the claims, comprise an acidifying particle or an amino carboxylic builder. Examples of an amino carboxylic builder include aminocarboxylic acids, salts and derivatives thereof. In some embodiment, the amino carboxylic builder is an aminopolycarboxylic builder, such as glycine-N,N-diacetic acid or derivative of general formula MOOC-CHR-N(CH₂COOM)₂ where R is C₁₋₁₂alkyl and M is alkali metal. In some embodiments, the amino carboxylic builder can be methylglycine diacetic acid (MGDA), GLDA (glutamic-N,N-diacetic acid), iminodisuccinic acid (IDS), carboxymethyl inulin and salts and derivatives thereof, aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl) aspartic acid (SMAS), N-(2-sulfoethyl)aspartic acid (SEAS), N-(2-sulfomethyl)glutamic acid (SMGL), N-(2-sulfoethyl) glutamic acid (SEGL), IDS (iminodiacetic acid) and salts and derivatives thereof such as N-methyliminodiacetic acid (MIDA), alpha-alanine-N,N-diacetic acid (alpha-ALDA), serine-N,N-diacetic acid (SEDA), isoserine-N,Ndiacetic acid (ISDA), phenylalanine-N,N-diacetic acid (PHDA), anthranilic acid- N,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts and derivative thereof. In some embodiments, the acidifying particle has a weight geometric mean particle size of from about 400 µ to about 1200 µ and a bulk density of at least 550 g/L. In some embodiments, the acidifying particle comprises at least about 5% of the builder.

In some embodiments, the acidifying particle can comprise any acid, including organic acids and mineral acids. Organic acids can have one or two carboxyls and in some instances up to 15 carbons, especially up to 10 carbons, such as formic, acetic, propionic, capric, oxalic, succinic, adipic, maleic, fumaric, sebacic, malic, lactic, glycolic, tartaric and glyoxylic acids. In some embodiments, the acid is citric acid. Mineral acids include hydrochloric and sulphuric acid. In some instances, the acidifying particle of the invention is a highly active particle comprising a high level of amino carboxylic builder. Sulphuric acid has been found to further contribute to the stability of the final particle.

In some embodiments, the cleaning compositions according to the present invention, as defined in the claims, comprise at least one surfactant and/or a surfactant system wherein the surfactant is selected from nonionic surfactants, anionic surfactants, cationic surfactants, ampholytic surfactants, zwitterionic surfactants, semi-polar nonionic surfactants and mixtures thereof. In some embodiments, the surfactant is present at a level of from about 0.1 to about 60%, while in alternative embodiments the level is from about 1 to about 50%, while in still further embodiments the level is from about 5 to about 40%, by weight of the cleaning composition.

In some embodiments, the cleaning compositions of the present invention, as defined in the claims, comprise one or more detergent builders or builder systems. In some embodiments incorporating at least one builder, the cleaning compositions comprise at least about 1%, from about 3 to about 60% or even from about 5 to about 40% builder by weight of the cleaning composition. Builders include, but are not limited to, the alkali metal, ammonium and alkanolammonium salts of polyphosphates, alkali metal silicates, alkaline earth and alkali metal carbonates, aluminosilicates, ether hydroxypolycarboxylates, copolymers ofmaleic anhydride with ethylene or vinyl methyl ether, 1,3,5-trihydroxybenzene-2,4,6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, and soluble salts thereof. Indeed, it is contemplated that any suitable builder will find use in the present invention.

In some embodiments, the builders form water-soluble hardness ion complexes (e.g., sequestering builders), such as citrates and polyphosphates (e.g., sodium tripolyphosphate and sodium tripolyphospate hexahydrate, potassium tripolyphosphate, and mixed sodium and potassium tripolyphosphate, etc.). It is contemplated that any suitable builder will find use in the compositions of the present invention, as defined in the claims, including those known in the art (See e.g., EP2100949).

In some embodiments, builders for use in compositions as defined in the claims include phosphate builders and non- phosphate builders. In some embodiments, the builder is a phosphate builder. In some embodiments, the builder is a non-phosphate builder. If present, builders are used in a level of from 0.1 to 80%, or from 5 to 60%, or from 10 to 50% by weight of the composition. In some embodiments the product comprises a mixture of phosphate and non-phosphate builders. Suitable phosphate builders include mono-phosphates, di-phosphates, tri-polyphosphates or oligomeric-poylphosphates, including the alkali metal salts of these compounds, including the sodium salts. In some embodiments, a builder can be sodium tripolyphosphate (STPP). Additionally, the composition can comprise carbonate and/or citrate, preferably citrate that helps to achieve a neutral pH composition of the invention. Other suitable non-phosphate builders include homopolymers and copolymers of polycarboxylic acids and their partially or completely neutralized salts, monomeric polycarboxylic acids and hydroxycarboxylic acids and their salts. In some embodiments, salts of the above mentioned compounds include the ammonium and/or alkali metal salts, i.e. the lithium, sodium, and potassium salts, including sodium salts. Suitable polycarboxylic acids include acyclic, alicyclic, hetero-cyclic and aromatic carboxylic acids, wherein in some embodiments, they can contain at least two carboxyl groups which are in each case separated from one another by, in some instances, no more than two carbon atoms.

In some embodiments, the cleaning compositions of the present invention, as defined in the claims, contain at least one chelating agent. Suitable chelating agents include, but are not limited to copper, iron and/or manganese chelating agents and mixtures thereof. In embodiments in which at least one chelating agent is used, the cleaning compositions of the present invention comprise from about 1.1 to about 15% or even from about 3.0 to about 10% chelating agent by weight of the subject cleaning composition.

In some still further embodiments, the cleaning compositions as defined in the claims contain at least one deposition aid. Suitable deposition aids include, but are not limited to, polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some compositions of the present invention as defined in the claims. In some embodiments, non-ionic surfactants find use. For example, in automatic dishwashing embodiments, non-ionic surfactants find use for surface modification purposes, in particular for sheeting, to avoid filming and spotting and to improve shine. These non-ionic surfactants also find use in preventing the redeposition of soils. In some embodiments, the anti-redeposition agent is a non-ionic surfactant as known in the art (See e.g., EP 2100949). In some embodiments, the non-ionic surfactant can be ethoxylated nonionic surfactants, epoxy-cappedpoly(oxyalkylated) alcohols and amine oxides surfactants.

In some embodiments, the cleaning compositions of the present invention, as defined in the claims, include one or more dye transfer inhibiting agents. Suitable polymeric dye transfer inhibiting agents include, but are not limited to, polyvinylpyrrolidone polymers, polyamine N-oxide polymers, copolymers ofN-vinylpyrrolidone and N-vinylimidazole, polyvinyloxazolidones and polyvinylimidazoles or mixtures thereof. In embodiments in which at least one dye transfer inhibiting agent is used, the cleaning compositions of the present invention comprise from about 0.0001 to about 10%, from about 0.01 to about 5%, or even from about 0.1 to about 3% by weight of the cleaning composition.

In some embodiments, silicates are included within the compositions of the present Invention, as defined in the claims. In some such embodiments, sodium silicates (e.g., sodium disilicate, sodium metasilicate, and crystalline phyllosilicates) find use. In some embodiments, silicates are present at a level of from about 1 to about 20%. In some embodiments, silicates are present at a level of from about 5 to about 15% by weight of the composition.

In some still additional embodiments, the cleaning compositions of the present invention also contain dispersants. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments, the enzymes used in the cleaning compositions, as defined in the claims, are stabilized by any suitable technique. In some embodiments, the enzymes employed herein are stabilized by the presence of water-soluble sources of calcium and/or magnesium ions in the finished compositions that provide such ions to the enzymes. In some embodiments, the enzyme stabilizers include oligosaccharides, polysaccharides, and inorganic divalent metal salts, including alkaline earth metals, such as calcium salts, such as calcium formate. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), Tin (II), cobalt (II), copper (II), nickel (II), and oxovanadium (IV). Chlorides and sulfates also find use in some embodiments of the present invention. Examples of suitable oligosaccharides and polysaccharides (e.g., dextrins) are known in the art (See e.g., WO07/145964). In some embodiments, reversible protease inhibitors also find use, such as boron-containing compounds (e.g., borate, 4-formyl phenyl boronic acid) and/or a tripeptide aldehyde find use to further improve stability, as desired.

In some embodiments, bleach, bleach activators and/or bleach catalysts are present in the compositions of the present invention. In some embodiments, the cleaning compositions of the present invention comprise inorganic and/or organic bleaching compound(s). Inorganic bleaches include, but are not limited to perhydrate salts (e.g., perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the present invention (See e.g., EP2100949).

In some embodiments, bleach activators are used in the compositions of the present invention. Bleach activators are typically organic peracid precursors that enhance the bleaching action in the course of cleaning at temperatures of 60°C and below. Bleach activators suitable for use herein include compounds which, under perhydrolysis conditions, give aliphatic peroxoycarboxylic acids having preferably from about 1 to about 10 carbon atoms, in particular from about 2 to about 4 carbon atoms, and/or optionally substituted perbenzoic acid. Additional bleach activators are known in the art and find use in the present invention (See e.g., EP2100949).

In addition, in some embodiments and as further described herein, the cleaning compositions of the present invention further comprise at least one bleach catalyst. In some embodiments, the manganese triazacyclononane and related complexes find use, as well as cobalt, copper, manganese, and iron complexes. Additional bleach catalysts find use in the present invention (See e.g., US 4,246,612; 5,227,084; and 4,810,410; and WO99/06521 and EP2100949).

In some embodiments, the cleaning compositions of the present invention contain one or more catalytic metal complexes. In some embodiments, a metal-containing bleach catalyst finds use. In some embodiments, the metal bleach catalyst comprises a catalyst system comprising a transition metal cation of defined bleach catalytic activity, (e.g., copper, iron, titanium, ruthenium, tungsten, molybdenum, or manganese cations), an auxiliary metal cation having little or no bleach catalytic activity (e.g., zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (See e.g., US 4,430,243). In some embodiments, the cleaning compositions of the present invention are catalyzed by means of a manganese compound. Such compounds and levels of use are well known in the art (See e.g., US 5,576,282). In additional embodiments, cobalt bleach catalysts find use in the cleaning compositions of the present invention. Various cobalt bleach catalysts are known in the art (See e.g., US 5,597,936 and 5,595,967) and are readily prepared by known procedures.

In some additional embodiments, the cleaning compositions of the present invention include a transition metal complex of a macropolycyclic rigid ligand (MRL). As a practical matter, and not by way of limitation, in some embodiments, the compositions and cleaning processes provided by the present invention are adjusted to provide on the order of at least one part per hundred million of the active MRL species in the aqueous washing medium, and in some embodiments, provide from about 0.005 to about 25 ppm, more preferably from about 0.05 to about 10 ppm, and most preferably from about 0.1 to about 5 ppm, of the MRL in the wash liquor.

In some embodiments, transition-metals in the instant transition-metal bleach catalyst include, but are not limited to manganese, iron and chromium. MRLs also include, but are not limited to special ultra-rigid ligands that are cross-bridged (e.g., 5,12-diethyl-1,5,8,12-tetraazabicyclo[6.6.2]hexadecane). Suitable transition metal MRLs are readily prepared by known procedures (See e.g., WO2000/32601, and US 6,225,464).

In some embodiments, the cleaning compositions of the present invention comprise metal care agents. Metal care agents find use in preventing and/or reducing the tarnishing, corrosion, and/or oxidation of metals, including aluminum, stainless steel, and non-ferrous metals (e.g., silver and copper). Suitable metal care agents include those described in EP2100949, WO9426860 and WO94/26859). In some embodiments, the metal care agent is a zinc salt. In some further embodiments, the cleaning compositions of the present invention comprise from about 0.1 to about 5% by weight of one or more metal care agent.

In some embodiments, the cleaning composition is a high density liquid (HDL) composition having a variant serine protease polypeptide protease. The HDL liquid laundry detergent can comprise a detersive surfactant (10-40%) comprising anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof); and optionally non-ionic surfactant (selected from a group of linear or branched or random chain, substituted or unsubstituted alkyl alkoxylated alcohol, for example a C₈-C₁₈alkyl ethoxylated alcohol and/or C₆-C₁₂alkyl phenol alkoxylates), optionally wherein the weight ratio of anionic detersive surfactant (with a hydrophilic index (HIc) of from 6.0 to 9) to non-ionic detersive surfactant is greater than 1:1.

The composition can comprise optionally, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05wt%-10wt%) and/or random graft polymers (typically comprising of hydrophilic backbone comprising monomers selected from the group consisting of: unsaturated C₁-C₆carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅alkyl group, polypropylene, polybutylene, vinyl ester of a saturated C₂-C₆mono-carboxylic acid, C₁-C₆alkyl ester of acrylic or methacrylic acid, and mixtures thereof.

The composition can comprise additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), anti-redeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition can further comprise saturated or unsaturated fatty acid, preferably saturated or unsaturated C₁₂-C₂₄fatty acid (0 to 10 wt%); deposition aids (e.g., polysaccharides, preferably cellulosic polymers, poly diallyl dimethyl ammonium halides (DADMAC), and co- polymers of DADMAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition can further comprise dye transfer inhibiting agents examples of which include manganese phthalocyanine, peroxidases, polyvinylpyrrolidone polymers, polyamine N-oxide polymers, copolymers ofN-vinylpyrrolidone and N-vinylimidazole, polyvinyloxazolidones and polyvinylimidazoles and/or mixtures thereof; chelating agents examples of which include ethylene-diamine-tetraacetic acid (EDTA); diethylene triamine penta methylene phosphonic acid (DTPMP); hydroxy-ethane diphosphonic acid (HEDP); ethylenediamine N,N'-disuccinic acid (EDDS); methyl glycine diacetic acid (MGDA); diethylene triamine penta acetic acid (DTPA); propylene diamine tetracetic acid (PDT A); 2-hydroxypyridine-N-oxide (HPNO); or methyl glycine diacetic acid (MGDA); glutamic acid N,N-diacetic acid (N,N-dicarboxymethyl glutamic acid tetrasodium salt (GLDA); nitrilotriacetic acid (NTA); 4,5-dihydroxy-m-benzenedisulfonic acid; citric acid and any salts thereof; N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), and derivatives thereof.

The composition of the invention may comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

The composition of the invention can further comprise silicone or fatty-acid based suds suppressors; heuing dyes, calcium and magnesium cations, visual signaling ingredients, antifoam (0.001 wt% to about 4.0wt%), and/or structurant/thickener (0.01 wt% to 5wt%, selected from the group consisting of diglycerides and triglycerides, ethylene glycol distearate, microcrystalline cellulose, cellulose based materials, micro fiber cellulose, biopolymers, xanthan gum, gellan gum, and mixtures thereof).

Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition of the invention can be any liquid form, for example a liquid or gel form, or any combination thereof. The composition may be in any unit dose form, for example a pouch.

In some embodiments, the cleaning composition is a high density powder (HDD) composition having a variant serine protease polypeptide protease. The HDD powder laundry detergent can comprise a detersive surfactant including anionic detersive surfactants (selected from a group of linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (selected from a group of linear or branched or random chain, substituted or unsubstituted C₈-C₁₈alkyl ethoxylates, and/or C₆-C₁₂alkyl phenol alkoxylates), cationic detersive surfactants (selected from a group of alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (selected from a group of alkanolamine sulpho-betaines); ampholytic surfactants; semi-polar non-ionic surfactants and mixtures thereof; builders (phosphate free builders [for example zeolite builders examples of which include zeolite A, zeolite X, zeolite P and zeolite MAP in the range of 0wt% to less than 10wt%]; phosphate builders [examples of which include sodium tri-polyphosphate in the range of 0 to less than 10wt%]; citric acid, citrate salts and nitrilotriacetic acid or salt thereof in the range of less than 15 wt%); silicate salt (sodium or potassium silicate or sodium meta-silicate in the range of 0 to less than 10wt%, or layered silicate (SKS-6)); carbonate salt (sodium carbonate and/or sodium bicarbonate in the range of 0 to less than 10 wt%); and bleaching agents (photobleaches, examples of which include sulfonated zinc phthalocyanines, sulfonated aluminum phthalocyanines, xanthenes dyes, and mixtures thereof; hydrophobic or hydrophilic bleach activators (examples of which include dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, and nonanoyloxybenzene sulfonate-NOBS, nitrile quats, and mixtures thereof; hydrogen peroxide; sources of hydrogen peroxide (inorganic perhydrate salts examples of which include mono or tetra hydrate sodium salt of perborate, percarbonate, persulfate, perphosphate, orpersilicate); preformed hydrophilic and/or hydrophobic peracids (selected from a group consisting of percarboxylic acids and salts, percarbonic acids and salts, perimidic acids and salts, peroxymonosulfuric acids and salts) & mixtures thereof and/or bleach catalyst (such as imine bleach boosters examples of which include iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof; metal-containing bleach catalyst for example copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid) and water-soluble salts thereof).

The composition of the invention can further comprise additional detergent ingredients including perfume microcapsules, starch encapsulated perfume accord, hueing agents, additional polymers including fabric integrity and cationic polymers, dye lock ingredients, fabric-softening agents, brighteners (for example C.I. Fluorescent brighteners), flocculating agents, chelating agents, alkoxylated polyamines, fabric deposition aids, and/or cyclodextrin.

In some embodiments, the cleaning composition is an ADW detergent composition having a serine protease of the present invention. The ADW detergent composition can comprise two or more non-ionic surfactants selected from a group of ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% comprising either phosphate (mono-phosphates, di-phosphates, tri-polyphosphates or oligomeric- poylphosphates, preferred sodium tripolyphosphate-STPP or phosphate-free builders [amino acid based compounds, examples of which include MGDA (methyl-glycine-diacetic acid), and salts and derivatives thereof, GLDA (glutamic-N,Ndiacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), B-alaninediacetic acid (B-ADA) and their salts], homopolymers and copolymers of polycarboxylic acids and their partially or completely neutralized salts, monomeric polycarboxylic acids and hydroxycarboxylic acids and their salts in the range of 0.5% to 50% by weight; sulfonated/carboxylated polymers (provide dimensional stability to the product) in the range of about 0.1 to about 50% by weight; drying aids in the range of about 0.1 to about 10% by weight (selected from polyesters, especially anionic polyesters optionally together with further monomers with 3 to 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1 to about 20%
by weight (sodium or potassium silicates for example sodium disilicate, sodium meta-silicate and crystalline phyllosilicates); bleach-inorganic (for example perhydrate salts such as perborate, percarbonate, perphosphate, persulfate and persilicate salts) and organic (for example organic peroxyacids including diacyl and tetraacylperoxides, especially diperoxydodecanedioc acid, diperoxytetradecanedioc acid, and diperoxyhexadecanedioc acid); bleach activators- organic peracid precursors in the range from about 0.1 to about 10% by weight; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1 to 5% by weight (selected from benzatriazoles, metal salts and complexes, and/or silicates); enzymes in the range from about 0.01 to 5.0mg of active enzyme per gram of automatic dishwashing detergent composition (acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, and any mixture thereof); and enzyme stabilizer components (selected from oligosaccharides, polysaccharides and inorganic divalent metal salts).

In some embodiments, the cleaning composition is borate-free. In some embodiments, the cleaning composition is phosphate-free.

Representative detergent formulations that beneficially include a serine protease polypeptide of the present invention include the detergent formulations found in WO2013063460, pages 78-152, and in particular the tables of pages 94 to 152. The serine proteases are normally incorporated into the detergent composition at a level of from 0.00001 to 10% of enzyme protein by weight of the composition. In some embodiments, the detergent composition comprises more than 0.0001%, 0.001%, 0.01%, or 0.1% of the serine protease by weight of the composition. In some embodiments, the detergent composition comprises less than 1%, 0.1%, 0.01%, or 0.001% of the serine protease by weight of the composition.

In some embodiments, removal of the oil or fat associated with raw feathers is assisted by using a serine protease polypeptide of the present invention. In some embodiments, the serine protease polypeptides are used in compositions for cleaning the feathers as well as to sanitize and partially dehydrate the fibers. In yet other embodiments, the disclosed serine protease polypeptides find use in recovering protein from plumage. In some other embodiments, the serine protease polypeptides are applied in a wash solution in combination with 95% ethanol or other polar organic solvent with a surfactant at about 0.5% (v/v).

In a further aspect of the invention, the serine protease polypeptides of the present invention can be used as a component of an animal feed composition, animal feed additive and/or pet food comprising a serine protease and variants thereof. The present invention further relates to a method for preparing such an animal feed composition, animal feed additive composition and/or pet food comprising mixing the serine protease polypeptide with one or more animal feed ingredients and/or animal feed additive ingredients and/or pet food ingredients. Furthermore, the present invention relates to the use of the serine protease polypeptide in the preparation of an animal feed composition and/or animal feed additive composition and/or pet food.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff" and "fodder" are used interchangeably and can comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; and e) minerals and vitamins.

In some embodiments, removal of the oil or fat associated with raw feathers is assisted by using a protease polypeptide of the present invention. In some embodiments, the protease polypeptides are used in compositions for cleaning the feathers as well as to sanitize and partially dehydrate the fibers. In some other embodiments, the protease polypeptides are applied in a wash solution in combination with 95% ethanol or other polar organic solvent with a surfactant at about 0.5% (v/v). In yet other embodiments, the disclosed protease polypeptides find use in recovering protein from plumage. The disclosed protease polypeptides may be used alone or in combination in suitable feather processing and proteolytic methods, such as those disclosed in PCT/EP2013/065362, PCT/EP2013/065363, and PCT/EP2013/065364. In some embodiments, the recovered protein can be subsequently used in animal or fish feed.

### EXAMPLES

The following examples are provided to demonstrate and illustrate certain preferred embodiments and aspects of the present disclosure and should not be construed as limiting.

In the experimental disclosure which follows, the following abbreviations apply: ADW (automatic dish washing); BMI (blood/milk/ink); BSA (bovine serum albumin); CAPS (*N-*cyclohexyl-3-aminopropanesulfonic acid); CHES (N-cyclohexyl-2-aminoethanesulfonic acid); DMC (dimethyl casein); HDD (heavy duty dry/powder); HDL (heavy duty liquid); HEPES (4-(2-hydroxyethyl)-1-piperazineethanesulfonic acid); MTP (microtiter plate); ND (not done); OD (optical density); PCR (polymerase chain reaction); ppm (parts per million); QS (quantity sufficient); rpm (revolutions per minute); AAPF (succinyl-Ala-Ala-Pro-Phe-p-nitroanilide); TNBSA (2,4,6-trinitrobenzene sulfonic acid); v/v (volume to volume); and w/v (weight to volume).

### EXAMPLE 1

### Discovery and Identification of Bacillus Serine Proteases

*Bacillus* sp. WDG290 and *Bacillus* sp. WDG291 (Dupont Culture Collection) were selected as a potential source for enzymes useful in industrial applications. To identify enzymes produced by these strains and the genes that encode these enzymes, the genomes of these strains were sequenced using Illumina® sequencing by synthesis (SBS) technology. Genome sequencing and assembly of the sequence data was performed by BaseClear (Leiden, The Netherlands). Contigs were annotated by BioXpr (Namur, Belgium). One of genes identified this way in strain *Bacillus* sp. WDG290 encodes a protein that shows homology to serine proteases of various other bacteria. The nucleotide sequence of this gene, *BspM04284.n,* is depicted in SEQ ID NO:1: TTGTTTAAAGGGAAATTATTTTCTAAAGTA ACAGCAGGGGTGCTCGCTGCAGCACTTCTCGCCGGAGGGACCGGCGCATATGCGGA CGCTGAGGACAGAAACGCAGGAGAAAAGAAGGAATACTTAATCGGATTCGAACCG GGTAAGGCAGCGGTCAACGCTTCCCAGATGATTACAGGTTTTGGCGGCGAAGTGGA ATACGAGTTTGAATTCGCCGATATTATGCACGTTACGCTGCCGGAACAGGCTCTGCA GGGGATCCAGAATAACCCGAACGTAGCGTTCATCGAAGAAAACTATGAAGCTGAAG CGTACAGCCAGACTGTGCCTTACGGAATCGAAAATATCGGTGCCGTTGATGTACAGC AGAATGACGGAAACACCGGAGACGGCGTAAGTGTCGCCGTTCTTGATACGGGCATT CAGCCTCATGAAGACTTAAACGTTCTTGGAGGCGCAAGCTTTATCTCAGACGAACCG ACATGGGAGGACTTCAACGGTCACGGGACACACGTAGCCGGTACAGTTGCAGCTCT AGATAATGATCTCGGAGTTCTCGGTGTGAGCCCTGACGTAGATCTTTACGCAGTGAA AGTGCTCGGTGCCAGTGGCGGCGGGTCCCACGCTTCCATCGCCCAGGGAATCGAAT GGGCCGTTCAGAACGATATGGACATCGTTAACATGAGCCTCGGCTCAAGCACAGGT TCCACAACTCTCCGTCAGGCAGTGGACTATGCCCACGGTGAAGGGGTTACCCTCGTG GCAGCAGCCGGCAACTCCGGTTCATTTCTGTTTTTGAATACGATCGGCTACCCGGCC AAATACGATAACGTAATCGCCGTTGGTGCGGTCGACGAAAATAACAGCCGCGCCTC GTTCTCCAGCGTCGGCAACCAGCTTGATGTGATGGCGCCCGGTGTCGCCACTCACAG CACTTACCTCGACAACAGCTACGCTTCCCTCAGCGGGACGAGCATGGCTTCCCCGCA TGTGGCCGGCGCTGCGGCACTGCTTCTTGCCCATAACCCGGTAGTCACAAACGAAGG GATCCGCGAGGCACTGAACAGCACCGCAATTCCGCTCGGCAGCCATTTCTATTACGG AAACGGTGTGATCGATGTCCGTGCCGCTTTAGAAGCGCAG.

The preproenzyme encoded by the *BspM04284.n* gene is depicted in SEQ ID NO:2. At the N-terminus, the protein has a signal peptide with a length of 30 amino acids as predicted by SignalP-NN. The presence of a signal peptide indicates that also this serine protease is a secreted enzyme. The enzyme has a pro sequence which is predicted to be 77 amino acids.

SEQ ID NO:2 sets forth the amino acid sequence of the serine protease precursor of BspM04284 (the signal peptide sequence is underlined and in bold, the prosequence is in italics):

LFKGKLFSKVTAGVLAAALLAGGTGAYADAEDRNAGEKKEYLIGFEPGKAAVNASQMI
TGFGGEVEYEFEFADIMHVTLPEQALQGIQNNPNVAFIEENYEAEAYSQTVPYGIENIGAVD
VQQNDGNTGDGVSVAVLDTGIQPHEDLNVLGGASFISDEPTWEDFNGHGTHVAGTVAA
LDNDLGVLGVSPDVDLYAVKVLGASGGGSHASIAQGIEWAVQNDMDIVNMSLGSSTGS
TTLRQAVDYAHGEGVTLVAAAGNSGSFLFLNTIGYPAKYDNVIAVGAVDENNSRASFSS
VGNQLDVMAPGVATHSTYLDNSYASLSGTSMASPHVAGAAALLLAHNPVVTNEGIREA
LNSTAIPLGSHFYYGNGVIDVRAALEAQ.

SEQ ID NO:3 sets forth the amino acid sequence of the predicted mature protease BspM04284 (276 amino acids): SQTVPYGIENIGAVDVQQNDGNTGDGVSVAVLDTGIQPH EDLNVLGGASFISDEPTWEDFNGHGTHVAGTVAALDNDLGVLGVSPDVDLYAVKVLG ASGGGSHASIAQGIEWAVQNDMDIVNMSLGSSTGSTTLRQAVDYAHGEGVTLVAAAG NSGSFLFLNTIGYPAKYDNVIAVGAVDENNSRASFSSVGNQLDVMAPGVATHSTYLDNS YASLSGTSMASPHVAGAAALLLAHNPVVTNEGIREALNSTAIPLGSHFYYGNGVIDVRA ALEAQ.

In *Bacillus* sp. WDG290, a second gene was identified encoding a serine protease. The nucleotide sequence of this gene, *BspM00695.n,* is depicted in SEQ ID NO:4: ATGGTCAG AACATCGAAGTTTAAAAAAGCAGCAGGTGCCGCACTGGCTGCCACACTTGTTTTTGG AGGAGCTGGCAGTATGATTCAGGCAGGAGACGGACCTGGTGAAACGAAAGAATACC TTGTCGGGTTCGAACCTGGGGCCGCAGCGAATGCGAACGGTGAAAATGCGGTCAGT GCACTTGGCGGTGAAGTGGAGCATGAGTTCGGGTTTGCGGATATTGTCCACGTGACA CTTCCTGAAGAAGCGGTACGAGGTCTTGAAAATAACCCTAATGTTGCCTACGTGGAA GAAAATGCGGAAGTGGAAGCTTACGCTCAGGAAGTGCCGTACGGGGTTGAGCAGAT CGGCGCACTTGATGTACAGCAGAACGACGGCAACACAGGAGCAGGCGTGAGCGTA GCCGTTCTTGATACAGGAATTGCAGACCATTCAGATCTGAATGTCGTCGGTGGTGCC AGTTTTGTAGCCGGTGAACCGGACTATGATGACTATAATGGTCATGGTACACATGTG GCTGGTACAGTCGCGGCCCTTGATAATGACTTCGGCGTTCTTGGCGTGAGTCCCGAT GTTGATCTATACGCGGTGAAGGTTCTTGGTGCAGACGGCAGTGGAACGATGGCCGG CATTGCCCAGGGAATTGAATGGGCAGCGGACAACGGGATGGACGTGGCGAATATGA GCCTTGGAGCTGATGTGGGGTCAACTACTCTCGAGCAGGCAGTGAACTATGCTCACAGTC AGGGTGTTACCTTAGTAGCCGCAGCCGGAAACTCCGGATCCCTCGGTCCATTTA ATACAATCGGTTACCCTGCAAAATACGACAATGTTATAGCAGTCGGTGCTGTTGACC AGAACAACGAACGGGCGTCCTTCTCCAGTGTCGGTGATGAACTGGACGTTATGGCTC CCGGTGTGAGCGTAAACAGCACATACTTGAATGATGGTTACCAGGCTCTTAATGGGA CATCCATGGCGGCACCTCACGTAGCCGGTGCAGCTGCCCTTATGCTTGCTGAACACC CTCATCTTTCCAACGATGATGTACGTAACGTATTTAACAGCACTGCCCAGCCGCTTG GCGATCACTTTTACTATGGCAACGGAGCGCTTGATGTAAGAGCAGCGCTTGATGCCC AG.

The preproenzyme encoded by the *BspM00695.n* gene is depicted in SEQ ID NO:5. At the N-terminus, the protein has a signal peptide with a length of 30 amino acids as predicted by SignalP-NN. The presence of a signal peptide indicates that this serine protease is also a secreted enzyme. The enzyme has a pro sequence which is predicted to be 75 amino acids.

SEQ ID NO:5 sets forth the amino acid sequence of the serine protease precursor of BspM00695 (the signal peptide sequence is underlined and in bold, the prosequence is in italics):

MVRTSKFKKAAGAALAATLVFGGAGSMIQAGDGPGETKEYLVGFEPGAAANANGENA
VSALGGEVEHEFGFADIVHVTLPEEAVRGLENNPNVAYVEENAEVEAYAQEVPYGVEQIGAL
DVQQNDGNTGAGVSVAVLDTGIADHSDLNVVGGASFVAGEPDYDDYNGHGTHVAGT
VAALDNDFGVLGVSPDVDLYAVKVLGADGSGTMAGIAQGIEWAADNGMDVANMSLG
ADVGSTTLEQAVNYAHSQGVTLVAAAGNSGSLGPFNTIGYPAKYDNVIAVGAVDQNNE
RASFSSVGDELDVMAPGVSVNSTYLNDGYQALNGTSMAAPHVAGAAALMLAEHPHLS

NDDVRNVFNSTAQPLGDHFYYGNGALDVRAALDAQ.

SEQ ID NO:6 sets forth the amino acid sequence of the predicted mature protease BspM00695 (276 amino acids): AQEVPYGVEQIGALDVQQNDGNTGAGVSVAVLDTGIAD HSDLNVVGGASFVAGEPDYDDYNGHGTHVAGTVAALDNDFGVLGVSPDVDLYAVKV LGADGSGTMAGIAQGIEWAADNGMDVANMSLGADVGSTTLEQAVNYAHSQGVTLVA AAGNSGSLGPFNTIGYPAKYDNVIAVGAVDQNNERASFSSVGDELDVMAPGVSVNSTY LNDGYQALNGTSMAAPHVAGAAALMLAEHPHLSNDDVRNVFNSTAQPLGDHFYYGN GALDVRAALDAQ.

In *Bacillus* sp. WDG291, another gene was identified encoding a serine protease. The nucleotide sequence of this gene, *BspZ00258.n,* is depicted in SEQ ID NO:7: ATGAAAAG ACATTCGTTTTTTAAGAAGGTAGCAGGTGCAGCCCTTGCTGCAACACTCGTTTTCGGAGG AGCAAGTGGATTTGTATCGGCGAACGGATCAGGAGAGACGAAGGAGTATTTGG TTGGTTTTCAACCTGGAGCAGCATCAGATAACGTGAATGCGTTAAGTCAGGTTACCG CTCTTGGTGGAGAGGTGGAGCATGAATTTGATTTTGCGGATATCATCCATGTTTCTTT ACCTGAACAAGCGGTTCGTGGTTTAGAAAACAATCCGAATGTAGCGTTTGTAGAAG AAAATCACGAAGTTCAAGCATATGCTCAAGAAGTCCCTTATGGTATTGAACAAATTG GCGCTATTGATGTACAGGTCAATGACGGAAACACAGGTAATGGCGTGAGTGTGGCT GTGTTGGATACGGGGATTGCCGCTCATGAGGATTTGAATGTGGTTGATGGTGCGAGT TTTATCGCAGGTGAACCTGATTACGAAGACTATAATGGTCATGGTACACACGTTGCT GGAACCGTCGCAGCCCTTGATAATGACTTAGGTGTTCTCGGTGTGAGCCCTGACGTT GATCTCTATGCTGTCAAAGTATTAGGTGCTGATGGGGGCGGTTCCCACGCATCCATT GCTCAAGGCATCGAGTGGGCGGTTGATAATAATATCGATGTTGTGAACATGAGTCTT GGTGGTGCTGTAGGTTCGACGACACTTGAGCAAGCAGTAAATTACGCTCATAGCCA AGGTGTAACCCTTATTGCAGCAGCCGGTAATGAAGGATCGCTTATTCCAGGTTTGAA CACGATCGGCTACCCAGCAAAGTATGATAATGTGATCGCGGTTGGGGCGGTTGACTC AAATAACAACCGTGCCTCGTTCTCGAGTGTTGGAAATGAGCTAGATGTGGTCGCACC AGGCGTGAGCATATTGAGTACCTACTTGGGCAATGACTACGCAGCGTTGAACGGTA CGTCTATGGCTTCTCCACACGTGGCAGGAGCTGCAGCGCTACTACTGGCAGAAAATC CAGGATTGACGAACGATCAAGTGCGCGCGGTATTTAACGAAACAGCCGTTCCACTG GGAGATCACTTCTACTATGGAAATGGATTAATTGATGTAAGAGCTGCGATTGATGCG CAG.

The preproenzyme encoded by the *BspZ00258.n* gene is depicted in SEQ ID NO:8. At the N-terminus, the protein has a signal peptide with a length of 30 amino acids as predicted by SignalP-NN. The presence of a signal peptide indicates that this serine protease is a secreted enzyme. The enzyme has a pro sequence which is predicted to be 75 amino acids.

SEQ ID NO:8 sets forth the amino acid sequence of the serine protease precursor of BspZ00258 (the signal peptide sequence is underlined and in bold, the prosequence is in italics):

MKRHSFFKKVAGAALAATLVFGGASGFVSANGSGETKEYLVGFQPGAASDNVNALSQV
TALGGEVEHEFDFADIIHVSLPEQAVRGLENNPNVAFVEENHEVQAYAQEVPYGIEQIGAID
VQVNDGNTGNGVSVAVLDTGIAAHEDLNVVDGASFIAGEPDYEDYNGHGTHVAGTVA
ALDNDLGVLGVSPDVDLYAVKVLGADGGGSHASIAQGIEWAVDNNIDVVNMSLGGAV
GSTTLEQAVNYAHSQGVTLIAAAGNEGSLIPGLNTIGYPAKYDNVIAVGAVDSNNNRASFSS
VGNELDVVAPGVSILSTYLGNDYAALNGTSMASPHVAGAAALLLAENPGLTNDQV
RAVFNETAVPLGDHFYYGNGLIDVRAAIDAQ.

SEQ ID NO:9 sets forth the amino acid sequence of the predicted mature protease BspZ00258 (277 amino acids): AQEVPYGIEQIGAIDVQVNDGNTGNGVSVAVLDTGIAAHE DLNVVDGASFIAGEPDYEDYNGHGTHVAGTVAALDNDLGVLGVSPDVDLYAVKVLGA DGGGSHASIAQGIEWAVDNNIDWNMSLGGAVGSTTLEQAVNYAHSQGVTLIAAAGNE GSLIPGLNTIGYPAKYDNVIAVGAVDSNNNRASFSSVGNELDWAPGVSILSTYLGNDY AALNGTSMASPHVAGAAALLLAENPGLTNDQVRAVFNETAVPLGDHFYYGNGLIDVR AAIDAQ.

### EXAMPLE 2

### Heterologous Expression of Bacillus sp. Serine Proteases

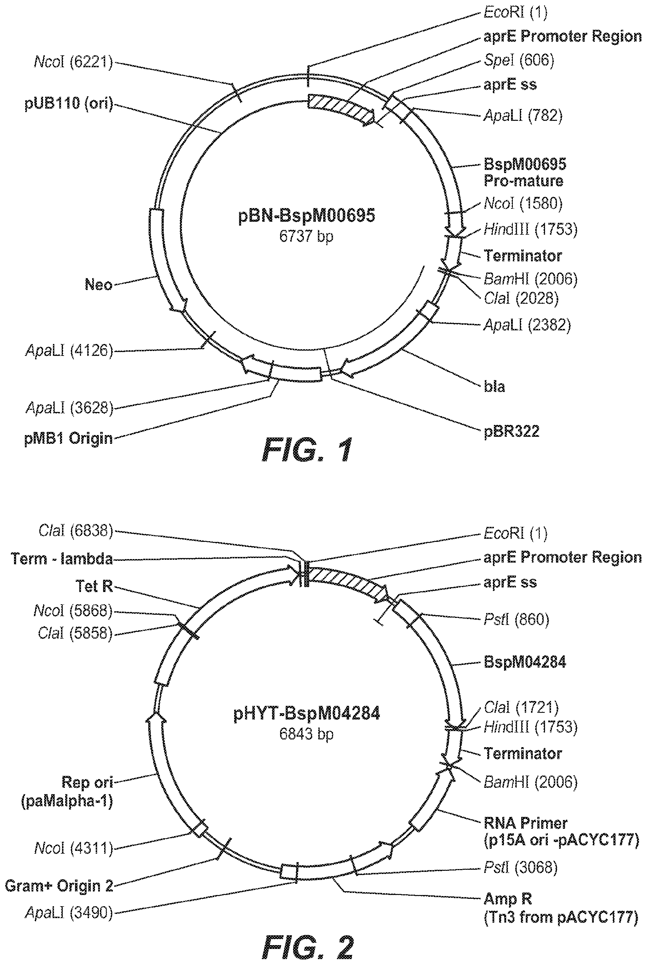

BspM04284 protease was produced in *B. subtilis* using an expression cassette consisting of the *B. subtilis aprE* promoter, the *B. subtilis aprE* signal peptide sequence, the native BspM04284 protease pro-peptide, the mature BspM04284 protease and a BPN' terminator. This cassette was cloned into the pHYT replicating shuttle vector and transformed into a suitable *B. subtilis* strain. The pHYT vector was derived from pHY300PLK (Takara) by adding a terminator after the tetracycline resistance gene using the *Bst*EII and *Eco*RI sites (terminator sequence, GGTTACCTTGAATGTATATAAACATTCTCAAAGGGATTTCTAAT AAAAAACGCTCGGTTGCCGCCGGGCGTTTTTTATGCATCGATGGAATTC) (SEQ ID NO:16). The *Hin*dIII site in pHY300PLK was also removed using a linker cloned into the BamHI and HindIII sites (new linker sequence, GGATCCTGACTGCCTGAGCTT) (SEQ ID NO:17). A map of the pHYT vector containing the BspM04284 gene (pHYT- BspM04284) is shown in Figure 1. To produce BspM04284, a *B. subtilis* transformant containing pHYT-BspM04284 was cultivated in an enriched semi-defined media based on MOPs buffer, with urea as major nitrogen source, glucose as the main carbon source, and supplemented with 1% soytone for robust cell growth. The media was supplemented with 25ppm tetracycline. After incubation (2 days at 32°C), BspM04284 protease was detected in the growth medium.

BspM00695 and BspZ00258 proteases were produced in *B. subtilis* using an expression cassette consisting of the *B. subtilis aprE* promoter, the *B. subtilis aprE* signal peptide sequence, the native protease pro-peptides, the mature gene of interest protease and a BPN' terminator. The cassettes were cloned into the pBN based replicating shuttle vector (Babe' et al. (1998), Biotechnol. Appl. Biochem. 27: 117-124) and a suitable *B. subtilis* strain was transformed with the vectors. A representative plasmid map of the pBN vector containing BspM00695 gene (pBN-BspM00695), is shown in Figure 2. To produce BspM00695 and BspZ00258, *B. subtilis* transformants containing pBN-BspM00695 and pBN-BspZ00258 were cultured in 15 ml Falcon tubes for 16 hours in TSB (broth) with 10 ppm neomycin, and 300 µl of the pre-cultures were added to a 500 mL flask filled with 30 mL of cultivation media (described below) supplemented with 10 ppm neomycin. The flasks were incubated for 48 hours at 32°C with constant rotational mixing at 180 rpm. Cultures were harvested by centrifugation at 14500 rpm for 20 min in conical tubes. The culture supernatants were used for assays. The cultivation media was an enriched semi-defined media based on MOPs buffer with urea as major nitrogen source, glucose as the main carbon source, and supplemented with 1% soytone for robust cell growth.

The nucleotide pro-mature sequence of the BspM04284 gene in plasmid pHYT-BspM04284 is depicted in SEQ ID NO:10:

GAGGACAGAAACGCAGGAGAAAAGAAGGAATACTTAATCGGATTCGAACCGGGTAA
GGCAGCGGTCAACGCTTCCCAGATGATTACAGGTTTTGGCGGCGAAGTGGAATACGA
GTTTGAATTTGCCGATATTATGCACGTTACGCTGCCGGAACAGGCTCTGCAGGGGA
TTCAGAATAACCCGAACGTAGCGTTCATCGAAGAAAACTATGAAGCTGAAGCGTA
CAGCCAGACTGTGCCTTACGGAATCGAAAATATCGGTGCCGTTGATGTTCAGCAGA
ATGACGGAAACACCGGAGACGGCGTAAGTGTCGCCGTTCTTGATACGGGCATTCA
GCCTCATGAAGACTTAAACGTTCTTGGAGGCGCATCATTTATCTCAGACGAACCGA
CATGGGAGGACTTCAACGGTCACGGACACACGTAGCCGGTACAGTTGCAGCTCT

AGATAATGATCTCGGAGTTCTCGGTGTGAGCCCTGACGTAGATCTTTACGCAGTGAA
AGTGCTCGGTGCCAGTGGCGGCGGGTCCCACGCTTCCATCGCCCAGGGAATCGAATG
GGCCGTTCAGAACGATATGGACATCGTTAACATGAGCCTCGGCTCAAGCACAGGTTC
CACAACTCTCCGTCAGGCAGTGGACTATGCCCACGGTGAAGGGGTTACCCTCGTGGC
AGCAGCCGGCAACTCCGGTTCATTTCTGTTTTTGAATACGATCGGCTACCCGGCCA
AATACGATAACGTAATCGCCGTTGGTGCGGTCGACGAAAATAACAGCCGCGCCTC
GTTCTCCAGCGTCGGCAACCAGCTTGATGTGATGGCGCCCGGTGTCGCCACTCACA
GCACTTACCTCGACAACAGCTACGCTTCCCTCAGCGGGACGAGCATGGCTTCCCCG
CATGTGGCCGGCGCTGCGGCACTGCTTCTTGCCCATAACCCGGTAGTCACAAACGA
AGGGATTCGCGAGGCACTGAACAGCACCGCAATTCCGCTCGGCAGCCATTTCTATTA
CGGAAACGGTGTGATCGATGTCCGTGCCGCTTTAGAAG CGCAG.

The amino acid sequence of the BspM04284 precursor protein expressed from plasmid pHYT-BspM04284 is depicted in SEQ ID NO:11 (the predicted pro-peptide is shown in underlined text): EDRNAGEKKEYLIGFEPGKAAVNASQMITGFGGEVEYEFEFADIMHVT LPEQALQGIQNNPNVAFIEENYEAEAYSQTVPYGIENIGAVDVQQNDGNTGDGVSVAVL DTGIQPHEDLNVLGGASFISDEPTWEDFNGHGTHVAGTVAALDNDLGVLGVSPDVDLY AVKVLGASGGGSHASIAQGIEWAVQNDMDIVNMSLGSSTGSTTLRQAVDYAHGEGVTL VAAAGNSGSFLFLNTIGYPAKYDNVIAVGAVDENNSRASFSSVGNQLDVMAPGVATHS TYLDNSYASLSGTSMASPHVAGAAALLLAHNPVVTNEGIREALNSTAIPLGSHFYYGNG VIDVRAALEAQ.

The nucleotide pro-mature sequence of the BspM00695 gene in plasmid pBN-BspM00695 is depicted in SEQ ID NO:12: GGAGACGGACCTGGTGAAACGAAAGAATAC CTTGTCGGGTTCGAACCTGGGGCCGCAGCGAATGCGAACGGTGAAAATGCGGTCAG TGCACTTGGCGGTGAAGTGGAGCATGAGTTCGGGTTTGCGGATATTGTCCACGTGAC ACTTCCTGAAGAAGCGGTACGAGGTCTTGAAAATAACCCTAATGTTGCCTACGTGGA AGAAAATGCGGAAGTGGAAGCATACGCTCAGGAAGTGCCGTACGGGGTTGAGCAG ATCGGCGCACTTGATGTTCAGCAGAACGACGGCAACACAGGAGCAGGCGTGAGCGT AGCCGTTCTTGATACAGGAATTGCAGACCATTCAGATCTGAATGTCGTCGGTGGTGC CAGTTTTGTAGCCGGTGAACCGGACTATGATGACTATAATGGTCATGGTACACATGT GGCTGGTACAGTCGCGGCCCTTGATAATGACTTCGGCGTTCTTGGCGTGAGTCCCGA TGTTGATCTATACGCGGTGAAGGTTCTTGGTGCAGACGGCAGTGGAACGATGGCCG GCATTGCCCAGGGAATTGAATGGGCAGCGGACAACGGGATGGACGTGGCGAATATG AGCCTTGGAGCTGATGTGGGGTCAACTACTCTGGAACAGGCAGTGAACTATGCTCAC AGTCAGGGTGTTACCTTAGTAGCCGCAGCCGGAAACTCCGGCTCACTCGGTCCATTT AATACAATCGGTTACCCTGCAAAATACGACAATGTTATAGCAGTCGGTGCTGTTGAC CAGAACAACGAACGGGCGTCCTTCTCCAGTGTCGGTGATGAACTGGACGTTATGGCT CCCGGTGTGAGCGTAAACAGCACATACTTGAATGATGGTTACCAGGCTCTTAATGGG ACATCCATGGCGGCACCTCACGTAGCCGGTGCAGCTGCCCTTATGCTTGCTGAACAC CCTCATCTTTCCAACGATGATGTACGTAACGTATTTAACAGCACTGCCCAGCCGCTT GGCGATCACTTTTACTATGGCAACGGAGCGCTTGATGTAAGAGCAGCGCTTGATGCCCAG TAA.

The amino acid sequence of the BspM00695 precursor protein expressed from plasmid pBN- BspM00695 is depicted in SEQ ID NO:13 (the predicted pro-peptide is shown in underlined text): GDGPGETKEYLVGFEPGAAANANGENAVSALGGEVEHEFGFADIVHV TLPEEAVRGLENNPNVAYVEENAEVEAYAQEVPYGVEQIGALDVQQNDGNTGAGVSV AVLDTGIADHSDLNVVGGASFVAGEPDYDDYNGHGTHVAGTVAALDNDFGVLGVSPD VDLYAVKVLGADGSGTMAGIAQGIEWAADNGMDVANMSLGADVGSTTLEQAVNYAH SQGVTLVAAAGNSGSLGPFNTIGYPAKYDNVIAVGAVDQNNERASFSSVGDELDVMAP GVSVNSTYLNDGYQALNGTSMAAPHVAGAAALMLAEHPHLSNDD.

The nucleotide pro-mature sequence of the BspZ00258 gene in plasmid pBN-BspZ00258 is depicted in SEQ ID NO:14: ACGGATCAGGAGAGACGAAGGAGTATTTGGT TGGTTTTCAACCTGGAGCAGCATCAGATAACGTGAATGCGTTAAGTCAGGTTACCGC TCTTGGTGGAGAGGTGGAGCATGAATTTGATTTTGCGGATATCATCCATGTTTCTTTA CCTGAACAAGCGGTTCGTGGTTTAGAAAACAATCCGAATGTAGCGTTTGTAGAAGA AAATCACGAAGTTCAAGCATATGCTCAAGAAGTCCCTTATGGTATTGAACAAATTGG CGCTATTGATGTCCAGGTCAATGACGGAAACACAGGTAATGGCGTGAGTGTGGCTG TGTTGGATACGGGGATTGCCGCTCATGAGGATTTGAATGTGGTTGATGGTGCGAGTT TTATCGCAGGTGAACCTGATTACGAAGACTATAATGGTCATGGTACACACGTTGCTG GAACCGTCGCAGCCCTTGATAATGACTTAGGTGTTCTCGGTGTGAGCCCTGACGTTG ATCTCTATGCTGTCAAAGTATTAGGTGCTGATGGGGGCGGTTCCCACGCATCCATTG CTCAAGGCATCGAGTGGGCGGTTGATAATAATATCGATGTTGTGAACATGAGTCTTG GTGGTGCTGTAGGTTCGACGACACTTGAGCAAGCAGTAAATTACGCTCATAGCCAA GGTGTAACCCTTATTGCAGCAGCCGGTAATGAAGGATCGCTTATTCCAGGTTTGAAC ACGATCGGCTACCCAGCAAAGTATGATAATGTGATCGCGGTTGGGGCGGTTGACTC AAATAACAACCGTGCCTCGTTCTCAAGTGTTGGAAATGAGCTAGATGTGGTCGCACC AGGCGTGAGCATATTGAGTACCTACTTGGGCAATGACTACGCAGCGTTGAACGGTA CGTCTATGGCTTCTCCACACGTGGCAGGAGCTGCAGCGCTACTACTGGCAGAAAATC CAGGATTGACGAACGATCAAGTGAGAGCGGTATTTAACGAAACAGCCGTTCCACTG GGAGATCACTTCTACTATGGAAATGGATTAATTGATGTAAGAGCTGCGATTGATGCG CAGTAA.

The amino acid sequence of the BspZ00258 precursor protein expressed from plasmid pBN-BspZ00258 is depicted in SEQ ID NO:15 (the predicted pro-peptide is shown in underlined text): NGSGETKEYLVGFQPGAASDNVNALSQVTALGGEVEHEFDFADIIHVS LPEQAVRGLENNPNVAFVEENHEVQAYAOEVPYGIEQIGAIDVQVNDGNTGNGVSVAV LDTGIAAHEDLNVVDGASFIAGEPDYEDYNGHGTHVAGTVAALDNDLGVLGVSPDVDL YAVKVLGADGGGSHASIAQGIEWAVDNNIDVVNMSLGGAVGSTTLEQAVNYAHSQGV TLIAAAGNEGSLIPGLNTIGYPAKYDNVIAVGAVDSNNNRASFSSVGNELDVVAPGVSIL STYLGNDYAALNGTSMASPHVAGAAALLLAENPGLTNDQVRAVFNETAVPLGDHFYY GNGLIDVRAAIDAQ.

### Protein Determination by Stain Free Imager Criterion

Protein was quantified by the stain-free Criterion™ TGX (Tris-Glycine extended) Stain-Free™ precast gels for PAGE which include unique trihalo compounds for rapid fluorescent detection of proteins with the Gel Doc™ EZ imaging system. Reagents used in the assay: Concentrated (10x) Laemmli Sample Buffer (Kem-En-Tec, Catalogue #42556); either 18 or 26-well Criterion TGX Strain-Free Precast gels (Bio-Rad, Catalogue #567-8124 and 567-8125, respectively); and protein markers (Bio-Rad, Catalogue #161- 0363). The assay was carried out as follow: 25µl protein sample and 25µl 0.5M HCL was added to a 96-well PCR plate on ice for 10 min to inactivate the protease and prevent self-hydrolysis. 50µl of the acid protein mix was added 50 µL sample buffer containing 0.385 mg DTT in the 96-wellPCR plate. After that the chamber was filled by running buffer, gel cassette was set. Then 10 µL of each sample together with markers was load in each pocket and electrophoresis was carried out at 200 V for 35 min. The gel was transferred to imager and Image Lab software was used for calculation of intensity of each band. Knowing the protein amount and the tryptophan content of the standard sample, the calibration curve can be made. The amount of experimental sample can be determined by extrapolation of the band intensity and tryptophan numbers to protein concentration. The protein quantification method was employed to prepare samples of the BspM04284, BspM00695, and BspZ00258 proteases used for the assays shown in the subsequent Examples.

### EXAMPLE 3

### Protease Activity of Bacillus sp. Serine Proteases

The protease activities of BspM04284, BspM00695, and BspZ00258 proteases were tested by measuring the hydrolysis of dimethyl casein (DMC) substrate. The reagent solutions used for the DMC assay were: 2.5% DMC (Sigma) in 100 mM Sodium Carbonate pH 9.5, 0.075% TNBSA (2,4,6-trinitrobenzene sulfonic acid, Thermo Scientific) in Reagent A. Reagent A: 45.4 g Na₂B₄O₇.10H₂O (Merck) in 15 mL 4N NaOH to reach a final volume of 1000 mL in MQ water, Dilution Solution: 10 mM NaCl, 0.1 mM CaCl₂, 0.005% Tween-80. Protease supernatants were diluted in Dilution Solution to appropriate concentration for the assay. A 96-well microtiter plate (MTP) was filled with 95 µl DMC substrate followed by the addition of 5 µl diluted protease supernatant. 100µL of TNBSA in Reagent A was then added with slow mixing. Activity was measured at 405 nm over 5 min using a SpectraMax plate reader in kinetic mode at RT. The absorbance of a blank containing no protease was subtracted from values. The activity was expressed as mOD/min. The protease activity measured for BspM04284, BspM00695, and BspZ00258 proteases are shown in Table 1.

| **Table 1: Protease activity of *Bacillus sp.* serine proteases on DMC substrate** | | |
|---|---|---|
| **Host Organism** | **Protease** | **Activity in DMC assay mOD/min/ppm** |
| *Bacillus* sp. WDG290 | BspM04284 | 50 |
| *Bacillus* sp. WDG290 | BspM00695 | 53 |
| *Bacillus* sp. WDG291 | BspZ00258 | 47 |
| *B. lentus* | GG36 | 54 |
| *B. amyloliquifaciens* | BPN' | 23 |

The pH dependence of proteolytic activity of *Bacillus sp.* serine proteases was studied using N-suc-AAPF-pNA (AAPF) as substrate in a 50mM Acetate/Bis-Tris/HEPES/CHES buffer. The activity was measured at pH between 4 to 11 with 1 pH unit increments. For the AAPF assay, the reagent solutions used were: 50 mM Acetate/Bis-Tris/HEPES/CHES buffer and 160 mM suc-AAPF-pNA in DMSO (suc-AAPF-pNA stock solution) (Sigma: S-7388). To prepare a working solution, 1 mL suc-AAPF-pNA stock solution was added to 100 mL Acetate/Bis-Tris/HEPES/CHES buffer and mixed. An enzyme sample was added to a MTP (Costar 9017) containing suc-AAPF-pNA working solution and assayed for activity at 405 nm over 3 min using a SpectraMax plate reader in kinetic mode at 40°C. The protease activity was expressed as mOD^{∗}min-1. The activity was converted to percentages of relative activity, by defining the activity at the optimal pH as 100%. Ranges for which the *Bacillus sp.* serine proteases maintain≥50% of activity under the conditions of this assay are shown in Table 2.

| **Table 2:pH Profile of *Bacillus sp.* Serine Proteases** | | |
|---|---|---|
| **Host Organism** | **Protease** | **pH range for which ≥50% activity is maintained** |
| *Bacillus* sp. WDG290 | BspM04284 | 9-11 |
| *Bacillus* sp. WDG290 | BspM00695 | 8-11 |
| *Bacillus* sp. WDG291 | BspZ00258 | 8-11 |

### EXAMPLE 4

### Comparison of Bacillus sp. Serine Proteases to Related Molecules

Related proteins were identified by a BLAST search (Altschul et al., Nucleic Acids Res, 25:3389-402, 1997) against the NCBI non-redundant protein database using the amino acid sequences of BspM04284 (SEQ ID NO:3), BspM00695 (SEQ ID NO:6), and BspZ00258 (SEQ ID NO:9) as query sequence and a subset are shown on Tables 2A-4A. A similar search was run against the Genome Quest Patent database with search parameters set to default values using the mature protein amino acid sequences for BspM04284 (SEQ ID NO:3), BspM00695 (SEQ ID NO:6), and BspZ00258 (SEQ ID NO:9) as the query sequence, and a subset are shown in Tables 2B-4B. Percent identity (PID) for both search sets is defined as the number of identical residues divided by the number of aligned residues in the pairwise alignment. Value labeled "Sequence length" on tables corresponds to the length (in amino acids) for the proteins referenced with the listed Accession numbers, while "Aligned length" refers to sequence used for alignment and PID calculation.

| **Table2A:List of sequences with percent identity to BspM04284 protein identified from the NCBI non-redundant protein database** | | | | |
|---|---|---|---|---|
| **Accession #** | **PID** | **Organism** | **Sequence Length** | **Alignment Length** |
| WP_026691049 | 78 | *Bacillus aurantiacus* | 383 | 277 |
| BAD21128.1 | 67 | *Bacillus sp. KSM-LD1 SB* | 377 | 277 |
| WP_010192405.1 | 67 | *Bacillus sp. m3-13* | 379 | 277 |
| AAC43581.1 | 67 | *Bacillus sp. SprD* | 379 | 277 |
| WP_023856571.1 | 64 | *Bacillus sp. CPSM8* | 378 | 275 |
| CAJ70731.1 | 64 | *Bacillus licheniformis* | 379 | 275 |
| AAT75303.1 | 64 | *Bacillus mojavensis* | 379 | 275 |
| WP_010192403.1 | 64 | *Bacillus sp. m3-13* | 381 | 276 |
| AAC43580.1 | 61 | *Bacillus sp. SprC* | 378 | 276 |
| BAD11988.2 | 61 | *Bacillus sp. KSM-LD1 SA* | 376 | 276 |
| ADK62564.1 | 59 | *Bacillus sp. B001* | 375 | 274 |

| **Table 2B: List of sequences with percent identity to BspM04284 protein identified from the Genome Quest database** | | | | |
|---|---|---|---|---|
| **Patent ID #** | **PID** | **Organism** | **Sequence Length** | **Alignment Length** |
| JP2004154003-0002 | 67.9 | *Bacillus sp. KSM-LD1* | 276 | 277 |
| JP2013500714-0412 | 64.0 | *Bacillus licheniformis* | 274 | 275 |
| JP2013500714-0403 | 64.0 | *Bacillus mojavensis* | 274 | 275 |
| WO2011014278 | 63.6 | *B. licheniformis ATCC 14580* | 274 | 275 |
| CN101215534-0002 | 63.6 | *Bacillus licheniformis YP1A* | 379 | 275 |
| WO9739130 | 63.6 | *B. licheniformis; strain PWD-1* | 379 | 275 |
| DE102005022145 | 63.6 | *B. licheniformis; strain DSM13* | 379 | 275 |
| JP2011155932 | 61.3 | *Bacillus sp. NKS-21* | 374 | 274 |

| **Table 3A: List of sequences with percent identity to BspM00695 protein identified from the NCBI non-redundant protein database** | | | | |
|---|---|---|---|---|
| **Accession #** | **PID** | **Organism** | **Sequence Length** | **Alignment Length** |
| WP 026691049 | 82 | Bacillus aurantiacus | 383 | 277 |
| WP_044803561 | 69 | Bacillus amyloliquefaciens | 333 | 230 |
| WP_043927109 | 68 | Bacillus licheniformis S 16 | 378 | 273 |
| WP_010192405.1 | 67 | *Bacillus sp. m3-13* | 379 | 276 |
| WP_023856571.1 | 67 | *Bacillus sp. CPSM8* | 378 | 273 |
| ABY65723.1 | 67 | *Bacillus subtilis* | 354 | 273 |
| CAJ70731.1 | 67 | *Bacillus licheniformis* | 379 | 274 |
| AAT75303.1 | 67 | *Bacillus mojavensis* | 379 | 273 |
| BAD21128.1 | 66 | *Bacillus sp. KSM-LD1 SB* | 377 | 276 |
| AAC43581.1 | 66 | *Bacillus sp. SprD* | 379 | 276 |
| WP_006636716.1 | 65 | *Bacillus sonorensis* | 378 | 273 |
| WP_010192403.1 | 62 | *Bacillus sp. m3-13* | 381 | 275 |
| AAC43580.1 | 62 | *Bacillus sp. SprC* | 378 | 275 |
| BAD11988.2 | 61 | *Bacillus sp. KSM-LD1 SA* | 376 | 275 |
| ADC49870.1 | 61 | *Bacillus pseudofirmus OF4* | 374 | 272 |
| BAA06158.1 | 61 | *Bacillus sp. ALP I* | 374 | 272 |
| ADK62564.1 | 60 | *Bacillus sp. B001* | 375 | 273 |
| AAA22212.1 | 59 | *Bacillus alkalophilus* | 380 | 272 |
| BAA02442.1 | 59 | *Bacillus sp.* | 380 | 272 |
| P29600 | 59 | *Bacillus lentus* | 269 | 272 |

| **Table 3B: List of sequences with percent identity to BspM00695 protein identified from the Genome Quest database** | | | | |
|---|---|---|---|---|
| **Patent ID #** | **PID** | **Organism** | **Sequence Length** | **Alignment Length** |
| EP405901-? | 68.1 | *Synthetic* | 274 | 273 |
| WO0071683 | 67.5 | *Bacillus licheniformis* | 275 | 274 |
| WO2011009859 | 67.8 | *Bacillus amyloliquefaciens* | 274 | 273 |
| CN101215534-0002 | 67.8 | *Bacillus licheniformis YP1A* | 379 | 273 |
| WO2011014278 | 67.4 | *B. licheniformis ATCC 14580* | 274 | 273 |
| WO2011014278-0103 | 67.4 | *Bacillus mojavensis* | 274 | 273 |
| DE102005022145 | 67.4 | *B. licheniformis; strain DSM13* | 379 | 273 |
| JP2004154003 | 66.7 | *Bacillus sp. KSM-LD1* | 276 | 276 |
| WO9739130 | 67.0 | *B. licheniformis; strain PWD-1* | 379 | 273 |
| JP2011155932 | 62.1 | *B. pseudofirmus; Strain FA30-01* | 374 | 272 |

| **Table 4A: List of sequences with percent identity to BspZ00258 protein identified from the NCBI non-redundant protein database** | | | | |
|---|---|---|---|---|
| **Accession #** | **PID** | **Organism** | **Sequence Length** | **Alignment Length** |
| WP_026691049 | 99.3 | *Bacillus aurantiacus* | 383 | 277 |
| KPB03395 | 68.5 | *Bacillus sp. CHD6a* | 379 | 276 |
| BAD21128.1 | 67.7 | *Bacillus sp. KSM-LD1 SB* | 377 | 276 |
| WP_051255158 | 67.4 | *Pontibacillus marinus* | 375 | 276 |
| AAC43581.1 | 67.4 | *Bacillus sp. SprD* | 379 | 276 |
| WP_052114905 | 67.0 | *Pontibacillus chungwhensis* | 385 | 276 |
| WP_047970559 | 66.7 | Bacillus sp. LL01 | 379 | 276 |
| WP_010192405.1 | 66.7 | *Bacillus sp. m3-13* | 379 | 276 |
| WP_048353666 | 66.1 | Bacillus sp. GO-13 | 378 | 274 |
| WP_043927109 | 65.7 | Bacillus licheniformis S 16 | 378 | 273 |
| WP_025811752 | 65.7 | Bacillus sp. KJ-16 | 378 | 274 |
| ABY65723.1 | 65.7 | *Bacillus subtilis* | 354 | 274 |
| AAT75303.1 | 65.7 | *Bacillus mojavensis* | 379 | 274 |
| CAJ70731.1 | 65.7 | *Bacillus licheniformis* | 379 | 274 |
| WP_006636716.1 | 63.5 | *Bacillus sonorensis* | 378 | 274 |
| WP_010192403.1 | 63.0 | *Bacillus sp. m3-13* | 381 | 276 |
| AAC43580.1 | 61.9 | *Bacillus sp. SprC* | 378 | 276 |
| BAD11988.2 | 60.9 | *Bacillus sp. KSM-LD1 SA* | 376 | 276 |
| ADK62564.1 | 59.8 | *Bacillus sp. B001* | 375 | 274 |

| **Table 4B:List of sequences with percent identity to BspZ00258 protein identified from the Genome Quest database** | | | | |
|---|---|---|---|---|
| **Patent ID #** | **PID** | **Organism** | **Sequence Length** | **Alignment Length** |
| JP2004154003-0002 | 67.8 | *Bacillus sp. KSM-LD1* | 377 | 276 |
| WO2015089441-0063 | 67.8 | *Bacillus sp. KSM-LD1* | 276 | 276 |
| WO2015089441-0065 | 67.4 | *Bacillus sp.* | 276 | 276 |
| WO2015038792-0025 | 66.7 | *Bacillus sp.* | 276 | 276 |
| | | | | |
| CN104232610-0001 | 65.7 | *B. licheniformis; BBE11-1 Synthetic* | 274 | 274 |
| JP2013500714-0112 | 65.7 | *Bacillus licheniformis* | 274 | 274 |
| WO2011014278-0103 | 65.7 | *Bacillus mojavensis* | 274 | 274 |
| CN101215534-0002 | 65.3 | *Bacillus licheniformis YP1A* | 379 | 274 |
| WO9739130-0002 | 65.3 | *B, licheniformis; strain PWD-1* | 379 | 274 |
| WO2011014278-0109 | 64.9 | *Bacillus licheniformis ATCC 14580* | 274 | 274 |
| DE102005022145-0032 | 65.0 | *Bacillus licheniformis; strain DSM13* | 379 | 274 |

### Alignment of Homologous Sequences

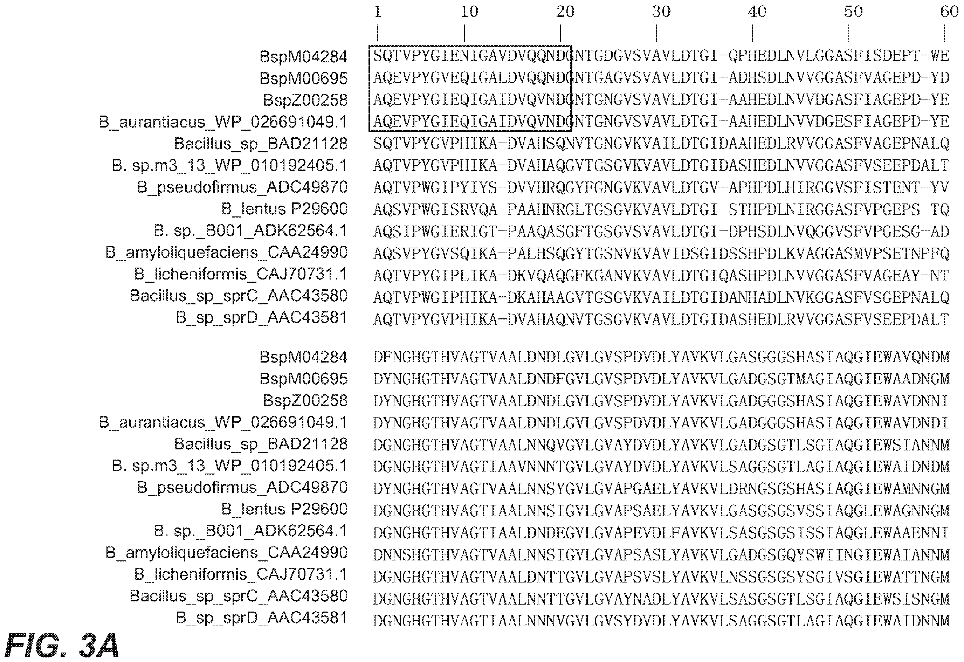

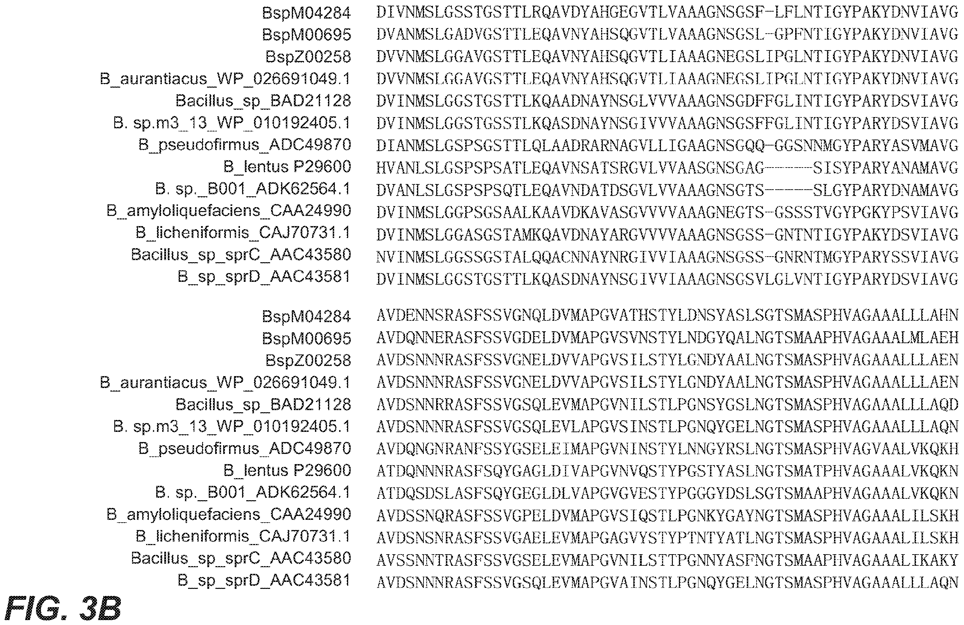

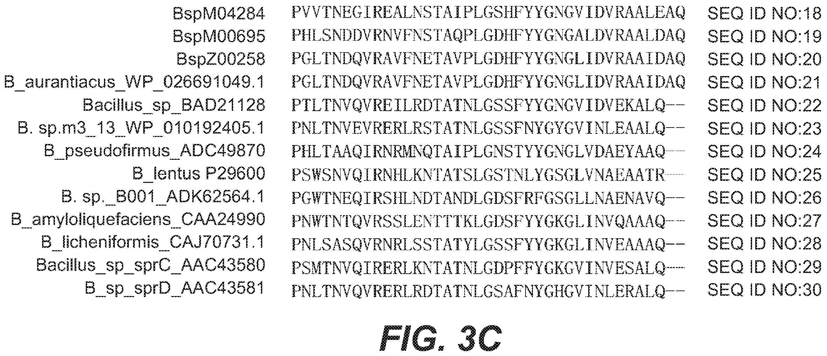

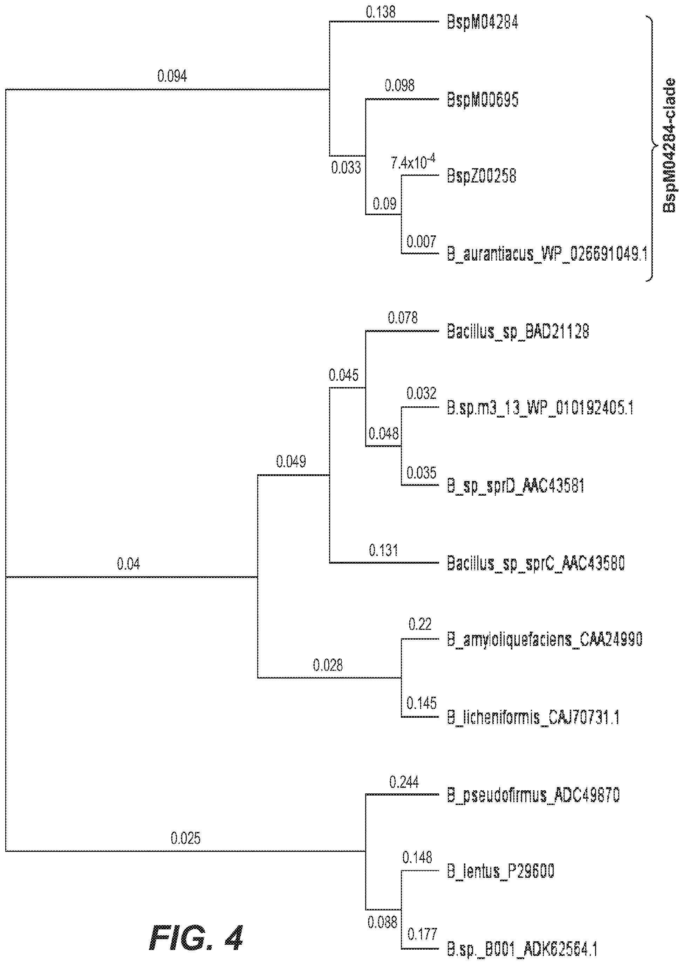

An alignment of the mature protein amino acid sequences for BspM04284 (SEQ ID NO:3), BspM00695 (SEQ ID NO:6), and BspZ00258 (SEQ ID NO:9) with the sequences of the mature forms of subtilisins from Tables 2A-4A is shown in Figure 3. The sequences were aligned with default parameters using the MUSCLE program from Geneious software (Biomatters Ltd.) (Robert C. Edgar. MUSCLE: multiple sequence alignment with high accuracy and high throughput Nucl. Acids Res. (2004) 32 (5): 1792-1797). A phylogenetic tree for amino acid sequences of the mature forms of the subtilisins from Figure 3 was built using the Geneious Tree builder program and is displayed in Figure 4.

### EXAMPLE 5

### Unique Features of the BspM04284-clade of Subtilisins

The Figure 3 alignment was reviewed for unique sequence similarities across the BspM04284-clade of subtilisins. The BspM04284-clade of subtilisins is characterized by a common motif over the sequence that begins at position 1 and ends at position 20, according to BspM04284 numbering. This motif can be characterized as XQXVPYGXEXIGAXDVQXND (SEQ ID NO:31), where X is any amino acid ("Motif 1"); or XQXVPYGXEXIGAXDVQXₐND (SEQ ID NO:32), where X is any amino acid and Xₐ is Q ("Motif 2"). Figure 2 includes a box around the motif.

The BspM04284, BspM00695, and BspZ00258 subtilisins, which have been identified as a BspM04284-clade of subtilisins based on the shared sequence motif set forth above, also cluster together in the phylogenetic tree that was built using various bacterial subtilisins and which is set forth in Figure 4.

The amino acid identity across the mature forms ofvarious subtilisins from Tables 2A-4A is shown on Table 7 below, wherein the percent amino acid identity is calculated over, for example, the 276 residues of the BspM04284 mature sequence.

**Table 7: Percent amino acid sequence identity**

| Mature enzyme | 1 | 2 | 3 | 4 | 5 | 6 | 7 | 8 | 9 | 10 | 11 | 12 | 13 |
|---|---|---|---|---|---|---|---|---|---|---|---|---|---|
| 1. BspM04284 | | 74.6 | 78.7 | 78.7 | 67.9 | 67.5 | 61.3 | 57.7 | 59.5 | 56.5 | 63.6 | 61.6 | 66.8 |
| 2. BspM00695 | 74.6 | | 82.7 | 82.3 | 66.4 | 66.8 | 61.3 | 59.1 | 60.6 | 59.8 | 66.9 | 62 | 66.1 |
| 3. BspZ00258 | 78.7 | 82.7 | | 99.3 | 67.5 | 66.4 | 61.1 | 58.2 | 59.3 | 60.6 | 64.9 | 61.7 | 67.1 |
| 4. WP_026691049.1 | 78.7 | 82.3 | 99.3 | | 66.8 | 66.4 | 61.1 | 57.8 | 58.9 | 59.9 | 64.5 | 61.4 | 66.4 |
| 5. BAD21128 | 67.9 | 66.4 | 67.5 | 66.8 | | 85.1 | 61.2 | 58 | 55.8 | 67.4 | 69.6 | 77.9 | 86.2 |
| 6. WP_010192405.1 | 67.5 | 66.8 | 66.4 | 66.4 | 85.1 | | 60.1 | 59.1 | 57.2 | 65.9 | 71.7 | 76.8 | 93.5 |
| 7. ADC49870 | 61.3 | 61.3 | 61.1 | 61.1 | 61.2 | 60.1 | | 62.3 | 57.5 | 56.4 | 62.4 | 61.8 | 60.9 |
| 8. P29600 | 57.7 | 59.1 | 58.2 | 57.8 | 58 | 59.1 | 62.3 | | 72.5 | 60 | 60.9 | 62.9 | 58 |
| 9. ADK62564.1 | 59.5 | 60.6 | 59.3 | 58.9 | 55.8 | 57.2 | 57.5 | 72.5 | | 56.4 | 58 | 58.5 | 56.9 |
| 10. CAA24990 | 56.5 | 59.8 | 60.6 | 59.9 | 67.4 | 65.9 | 56.4 | 60 | 56.4 | | 69.5 | 65.1 | 65.2 |
| 11. CAJ70731.1 | 63.6 | 66.9 | 64.9 | 64.5 | 69.6 | 71.7 | 62.4 | 60.9 | 58 | 69.5 | | 70.9 | 70.3 |
| 12. AAC43580 | 61.6 | 62 | 61.7 | 61.4 | 77.9 | 76.8 | 61.8 | 62.9 | 58.5 | 65.1 | 70.9 | | 77.2 |
| 13. AAC43581 | 66.8 | 66.1 | 67.1 | 66.4 | 86.2 | 93.5 | 60.9 | 58 | 56.9 | 65.2 | 70.3 | 77.2 | |

## Claims

1. A subtilisin or recombinant polypeptide, or active fragment thereof, wherein said subtilisin, polypeptide or fragment comprises a XQXVPYGXEXIGAXDVQQND (SEQ ID NO:32) motif, wherein X is any amino acid, and wherein said subtilisin, polypeptide or fragment comprises an amino acid sequence having at least 85% amino acid sequence identity to the amino acid sequence SEQ ID NO: 3 or 6, and wherein said subtilisin, polypeptide or fragment has protease activity.

2. The subtilisin, polypeptide or fragment of Claim 1, wherein the protease activity is a subtilisin protease activity on a dimethylcasein substrate.

3. A composition comprising: i) a surfactant; and ii) the subtilisin, polypeptide or fragment of any one of Claims 1-2.

4. The composition of Claim 3, wherein the surfactant is selected from the group consisting of an anionic surfactant, a cationic surfactant, a zwitterionic surfactant, an ampholytic surfactant, a semi-polar non-ionic surfactant, and a combination thereof.

5. The composition of any one of Claims 3-4, wherein:
a) said composition further comprises at least one calcium ion and/or zinc ion; at least one stabilizer; from about 0.001 % to about 1.0 weight % of said subtilisin, polypeptide or fragment of any one of Claims 1-2; at least one bleaching agent; at least one adjunct ingredient; and/or one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, additional serine proteases, and combinations thereof;
b) said composition contains phosphate or is phosphate-free and/or contains borate or is borate-free; and/or
c) said composition is formulated at a pH of from 8 to 12.

6. A method of cleaning, comprising: contacting a surface or an item in need of cleaning with the subtilisin, polypeptide or fragment of any one of Claims 1-2 or the composition of any one of Claims 3-5; and optionally further comprising the step of rinsing said surface or item after contacting said surface or item with said subtilisin, recombinant polypeptide, or fragment thereof, or after contacting said surface or item with said composition.

7. A polynucleotide comprising a nucleic acid sequence encoding the subtilisin, polypeptide or fragment of any one of Claims 1-2.

8. The polynucleotide of Claim 7, wherein said polynucleotide comprises a nucleic acid sequence having at least 85% identity to the nucleic acid sequence of SEQ ID NO:1 or 4.

9. An expression vector comprising the polynucleotide of Claim 7 or 8.

10. A host cell comprising the vector of Claim 9.

11. The host cell of Claim 10, wherein the host cell is of a species selected from *Bacillus spp*., *Streptomyces spp*., *Escherichia spp., Aspergillus spp*., *Trichoderma spp*., *Pseudomonas spp*., *Corynebacterium spp*., *Saccharomyces spp*., and *Pichia spp.*

12. A method for producing the subtilisin, polypeptide or fragment of any one of Claims 1-2, comprising:
(a) stably transforming the host cell of any one of Claims 10-11 with the expression vector of Claim 9;
(b) cultivating said transformed host cell under conditions suitable for said host cell to produce said subtilisin or polypeptide; and
(c) recovering said subtilisin or polypeptide.

13. The method of Claim 12, wherein said expression vector comprises a heterologous polynucleotide sequence encoding a heterologous pro-peptide, or one or both of a heterologous promoter and a polynucleotide sequence encoding a heterologous signal peptide.

## Patentansprüche

1. Subtilisin oder rekombinantes Polypeptid oder aktives Fragment davon, wobei das Subtilisin, Polypeptid oder Fragment ein XQXVPYGXEXIGAXDVQQND- (SEQ ID NO:32-) Motiv umfasst, wobei X irgendeine Aminosäure ist und wobei das Subtilisin, Polypeptid oder Fragment eine Aminosäuresequenz umfasst, die mindestens 85% Aminosäuresequenzidentität mit der Aminosäuresequenz SEQ ID NO: 3 oder 6 aufweist und wobei das Subtilisin, Polypeptid oder Fragment Proteaseaktivität aufweist.

2. Subtilisin, Polypeptid oder Fragment nach Anspruch 1, wobei die Proteaseaktivität eine Subtilisinproteaseaktivität auf einem Dimethylcaseinsubstrat ist.

3. Zusammensetzung umfassend: i) ein Tensid; und ii) das Subtilisin, Polypeptid oder Fragment nach einem der Ansprüche 1-2.

4. Zusammensetzung nach Anspruch 3, wobei das Tensid aus der Gruppe ausgewählt wird bestehend aus einem anionischen Tensid, einem kationischen Tensid, einem zwitterionischen Tensid, einem ampholytischen Tensid, einem semipolaren nichtionischen Tensid und einer Kombination davon.

5. Zusammensetzung nach einem der Ansprüche 3-4, wobei:
(a) die Zusammensetzung ferner Folgendes umfasst: mindestens ein Calciumion und/oder Zinkion, mindestens einen Stabilisator; etwa 0,001 bis etwa 1,0 Gew.-% des Subtilisins, Polypeptids oder Fragments nach einem der Ansprüche 1-2; mindestens ein Bleichmittel; mindestens einen Zusatzbestandteil; und/oder ein mehrere zusätzliche Enzyme oder Enzymderivate ausgewählt aus der Gruppe bestehend aus Acyltransferasen, Alpha-Amylasen, Beta-Amylasen, Alpha-Galactosidasen, Arabinosidasen, Arylesterasen, Beta-Galactosidasen, Carrageenasen, Catalasen, Cellobiohydrolasen, Cellulasen, Chondroitinasen, Cutinasen, Endo-beta-1,4-glucanasen, Endo-beta-mannanasen, Esterasen, Exomannanasen, Galactanasen, Glucoamylasen, Hemicellulasen, Hyaluronidasen, Keratinasen, Laccasen, Lactasen, Ligninasen, Lipasen, Lipoxygenasen, Mannanasen, Oxidasen, Pectatlyasen, Pectinacetylesterasen, Pectinasen, Pentosanasen, Peroxidasen, Phenoloxidasen, Phosphatasen, Phospholipasen, Phytasen, Polygalacturonasen, Proteasen, Pullulanasen, Reductasen, Rhamnogalacturonasen, Beta-Glucanasen, Tannasen, Transglutaminasen, Xylanacetylesterasen, Xylanasen, Xyloglucanasen, Xylosidasen, Metalloproteasen, zusätzlichen Serinproteasen und Kombinationen davon;
(b) die Zusammensetzung Phosphat enthält oder phosphatfrei ist und/oder Borat enthält oder boratfrei ist; und/oder
(c) die Zusammensetzung bei einem pH-Wert von 8 bis 12 formuliert ist.

6. Reinigungsverfahren umfassend: Kontaktieren einer Oberfläche oder eines Gegenstands, die/der des Reinigens bedarf, mit dem Subtilisin, Polypeptid oder Fragment nach irgendeinem der Ansprüche 1-2 oder der Zusammensetzung nach irgendeinem der Ansprüche 3-5; und wahlweise ferner den Schritt umfassend des Spülens der Oberfläche oder des Gegenstands nach Kontaktieren der Oberfläche oder des Gegenstands mit dem Subtilisin, rekombinanten Polypeptid oder Fragment davon oder nach Kontaktieren der Oberfläche oder des Gegenstands mit der Zusammensetzung.

7. Polynukleotid umfassend eine Nukleinsäuresequenz, die für das Subtilisin, Polypeptid oder Fragment nach einem der Ansprüche 1-2 codiert.

8. Polynukleotid nach Anspruch 7, wobei das Polynukleotid eine Nukleinsäuresequenz umfasst, die mindestens 85% Identität mit der Nukleinsäuresequenz von SEQ ID NO:1 oder 4 aufweist.

9. Expressionsvektor umfassend das Polynukleotid nach Anspruch 7 oder 8.

10. Wirtszelle umfassend den Vektor nach Anspruch 9.

11. Wirtszelle nach Anspruch 10, wobei die Wirtszelle eine Spezies ist ausgewählt unter *Bacillus spp., Streptomyces spp*., *Escherichia spp., Aspergillus spp*., *Trichoderma spp*., *Pseudomonas spp*., *Corynebacterium spp., Saccharomyces spp*., und *Pichia spp.*

12. Verfahren zum Herstellen des Subtilisins, Polypeptids oder Fragments nach einem der Ansprüche 1-2, umfassend:
(a) stabil Transformieren der Wirtszelle nach einem der Ansprüche 10-11 mit dem Expressionsvektor nach Anspruch 9;
(b) Züchten der transformierten Wirtszelle unter Bedingungen, die geeignet sind, dass die Wirtszelle das Subtilisin oder Polypeptid erzeugt; und
(c) Gewinnen des Subtilisins oder Polypeptids.

13. Verfahren nach Anspruch 12, wobei der Expressionsvektor eine heterologe Polynukleotidsequenz, die für ein heterologes Pro-Peptid codiert, oder eines oder beide von einem heterologen Promotor und einer Polynukleotidsequenz, die für ein heterologes Signalpeptid codiert, umfasst.

## Revendications

1. Subtilisine ou un polypeptide recombinant, ou un fragment actif de celui-ci, où ladite subtilisine, ledit polypeptide ou ledit fragment comprend un motif XQXVPYGXEXIGAXDVQQND (SEQ ID NO: 32), où X est tout acide aminé et où ladite subtilisine, ledit polypeptide ou ledit fragment comprend une séquence d'acides aminés ayant au moins 85% d'identité de séquence d'acides aminés avec la séquence d'acides aminés SEQ ID NO: 3 ou 6 et où ladite subtilisine, ledit polypeptide ou ledit fragment a une activité de protéase.

2. Subtilisine, polypeptide ou fragment selon la revendication 1, où l'activité de protéase est une activité de protéase de subtilisine sur un substrat de diméthylcaséine.

3. Composition comprenant: i) un tensioactif; et ii) la subtilisine, le polypeptide, ou le fragment selon l'une quelconque des revendications 1-2.

4. Composition selon la revendication 3, dans laquelle le tensioactif est choisi dans le groupe constitué de: un tensioactif anionique, un tensioactif cationique, un tensioactif zwitterionique, un tensioactif ampholytique, un tensioactif non ionique semi-polaire et une combinaison de ceux-ci.

5. Composition selon l'une quelconque des revendications 3-4, dans laquelle:
a) ladite composition comprend en outre au moins un ion calcium et/ou un ion zinc; au moins un stabilisant; d'environ 0,001% à environ 1,0% en poids de ladite subtilisine, dudit polypeptide ou dudit fragment selon l'une quelconque des revendications 1-2; au moins un agent de blanchiment; au moins un ingrédient adjuvant; et/ou un ou plusieurs enzymes ou dérivés d'enzymes supplémentaires choisis dans le groupe constitué de: acyltransférases, alpha-amylases, bêta-amylases, alpha-galactosidases, arabinosidases, arylestérases, bêta-galactosidases, carraghénases, catalases, cellobiohydrolases, cellulases, chondroïtinases, cutinases, endo-bêta-1,4-glucanases, endo-bêta-mannanases, estérases, exo-mannanases, galactanases, glucoamylases, hémicellulases, hyaluronidases, kératinases, laccases, lactases, ligninases, lipases, lipoxygénases, mannanases, oxydases, pectate lyases, pectine acétylestérases, pectinases, pentosanases, peroxydases, phénoloxydases, phosphatases, phospholipases, phytases, polygalacturonases, protéases, pullulanases, réductases, rhamnogalacturonases, bêta-glucanases, tannases, transglutaminases, xylane acétyl-estérases, xylanases, xyloglucanases, xylosidases, métalloprotéases, sérine protéases supplémentaires et des combinaisons de celles-ci;
b) ladite composition contient du phosphate ou est sans phosphate et/ou contient du borate ou est sans borate; et/ou
c) ladite composition est formulée à un pH de 8 à 12.

6. Méthode pour un nettoyage, comprenant: la mise en contact d'une surface ou d'un article ayant besoin d'un nettoyage avec la subtilisine, le polypeptide ou le fragment selon l'une quelconque des revendications 1-2 ou la composition selon l'une quelconque des revendications 3-5; et optionnellement comprenant en outre l'étape de rinçage de ladite surface ou dudit article après la mise en contact de ladite surface ou dudit article avec ladite subtilisine, ledit polypeptide recombinant ou ledit fragment de celui-ci, ou après la mise en contact de ladite surface ou dudit article avec ladite composition.

7. Polynucléotide comprenant une séquence d'acides nucléiques codant pour la subtilisine, le polypeptide ou le fragment selon l'une quelconque des revendications 1-2.

8. Polynucléotide selon la revendication 7, où ledit polynucléotide comprend une séquence d'acides nucléiques ayant au moins 85% d'identité avec la séquence d'acides nucléiques de la SEQ ID NO: 1 ou 4.

9. Vecteur d'expression comprenant le polynucléotide selon la revendication 7 ou 8.

10. Cellule hôte comprenant le vecteur selon la revendication 9.

11. Cellule hôte selon la revendication 10, où la cellule hôte est d'une espèce choisie parmi: *Bacillus spp., Streptomyces spp*., *Escherichia spp*., *Aspergillus spp., Trichoderma spp*., *Pseudomonas spp*., *Corynebacterium spp., Saccharomyces spp.* et *Pichia spp.*

12. Méthode pour la production de la subtilisine, du polypeptide ou du fragment selon l'une quelconque des revendications 1-2, comprenant:
(a) la transformation stable de la cellule hôte selon l'une quelconque des revendications 10-11 avec le vecteur d'expression selon la revendication 9;
(b) la culture de ladite cellule hôte transformée dans des conditions appropriées pour que ladite cellule hôte produise ladite subtilisine ou ledit polypeptide; et
(c) la récupération de ladite subtilisine ou dudit polypeptide.

13. Méthode selon la revendication 12, dans laquelle ledit vecteur d'expression comprend une séquence de polynucléotide hétérologue codant pour un pro-peptide hétérologue, ou un ou les deux parmi un promoteur hétérologue et une séquence de polynucléotide codant pour un peptide signal hétérologue.
